# EUROPEAN PATENT APPLICATION

(11) **EP 4 674 923 A1**
(43) Date of publication of application: **07.01.2026**
(21) Application number: 24763903.2
(22) Date of filing: 27.02.2024
(51) Int. Cl.: C09K 9/02, C07D 311/78, G02C 7/10

(54) **PHOTOCHROMIC COMPOUND, PHOTOCHROMIC COMPOSITION, PHOTOCHROMIC ARTICLE, AND EYEGLASSES**

(30) Priority: 28.02.2023 JP 2023030189
(71) Applicant: Hoya Lens Thailand Ltd., Pathumthani 12130 (TH)
(72) Inventor: KAWAKAMI, Hironori, Tokyo 160-8347 (JP); KOBAYASHI, Kei, Tokyo 160-8347 (JP)
(74) Representative: Hoffmann Eitle
(86) International application number: PCT/JP2024/007022
(87) International publication number: WO 2024/181424

(57) **Abstract**

A photochromic compound represented by General Formula 1 is provided. In General Formula 1, A represents a ring structure having 3 or more carbon atoms constituting a ring including the carbon atom at position 13 of indeno-fused naphthopyran, R¹ and R⁴ to R⁸ each independently represent a hydrogen atom or a substituent, R² represents an electron-donating group (excluding a sulfur-containing electron-donating group), R³ represents a hydrogen atom or an electron-donating group (excluding a sulfur-containing electron-donating group), and B and B' each independently represent a substituent).

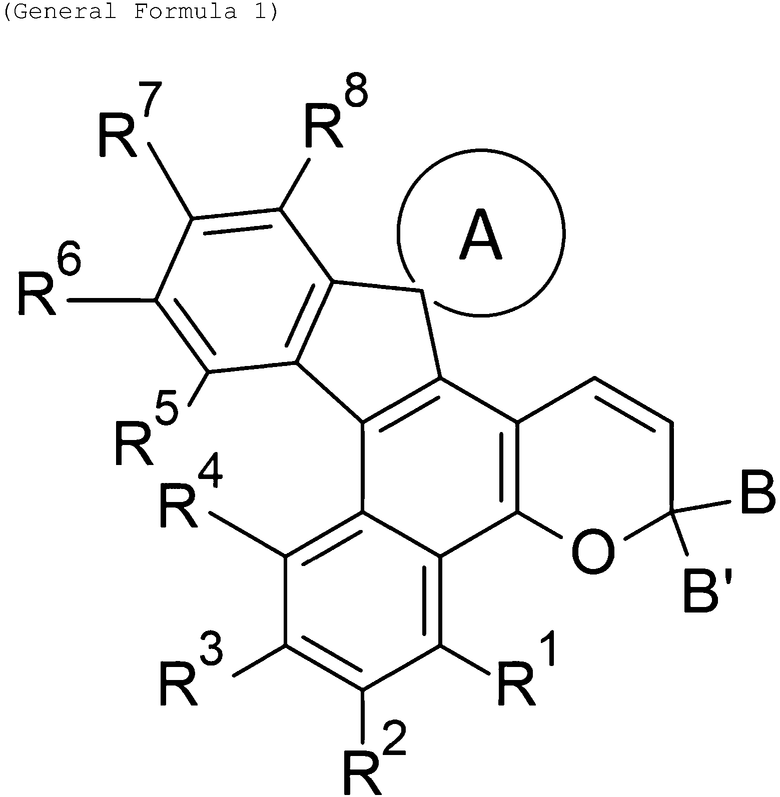

## Description

### [Technical Field]

The present invention relates to a photochromic compound, a photochromic composition, a photochromic article and spectacles.

### [Background Art]

A photochromic compound is a compound having a property of coloring under emission of light in a wavelength range for which it has photoresponsivity and fading without light emission (photochromic properties). For example, PTL 1 discloses a naphthopyran compound having photochromic properties.

### [Citation List]

### [Patent Literature]

[PTL 1] WO 2000/15631

### [Summary of Invention]

### [Technical Problem]

As a method of imparting photochromic properties to an article such as a spectacle lens, a method of incorporating a photochromic compound into a substrate and a method of forming a layer containing a photochromic compound may be exemplified.

Examples of performance desired for articles to which photochromic properties are imparted in this manner include a small molar absorption coefficient at least one wavelength in the visible range (a wavelength of 380 to 780 nm) when no light is emitted (that is, high transparency in the visible range) and a fast fading rate after coloring according to light emission.

An object of one aspect of the present invention is to provide a photochromic article that has high transparency in the visible range when uncolored and exhibits a fast fading rate after coloring.

### [Solution to Problem]

One aspect of the present invention relates to a photochromic compound represented by the following General Formula 1.

In addition, one aspect of the present invention relates to a photochromic article including one or more photochromic compounds represented by the following General Formula 1.

In addition, one aspect of the present invention relates to a photochromic composition including one or more photochromic compounds represented by the following General Formula 1.

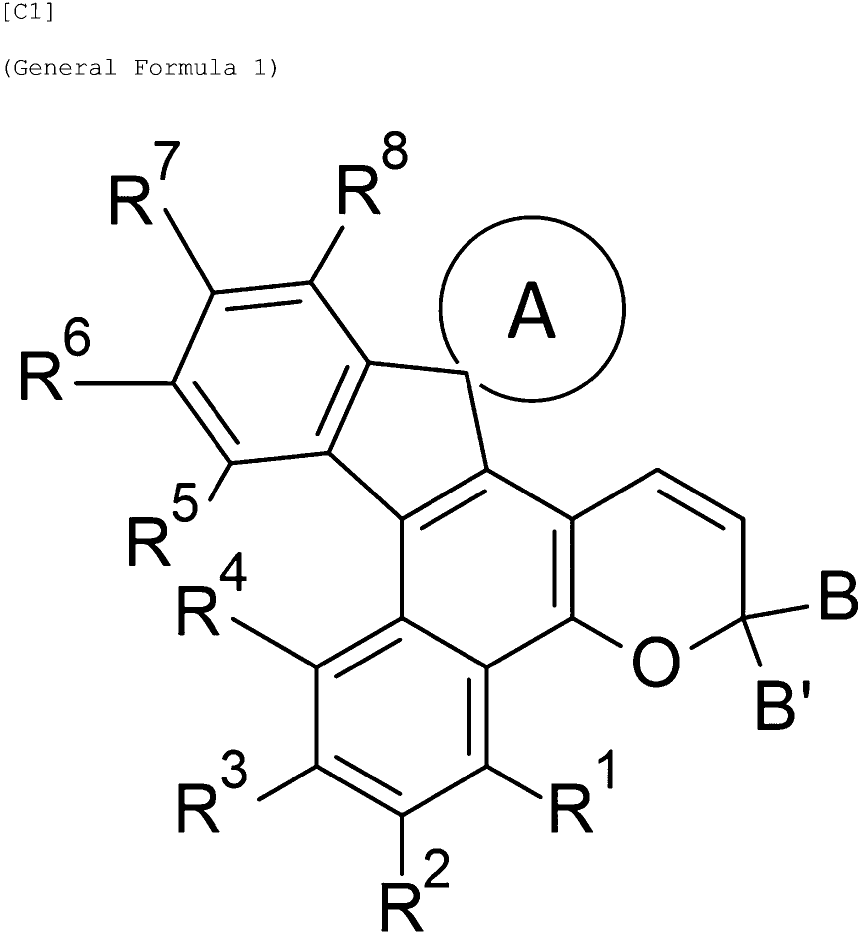

In General Formula 1,
A represents a ring structure having 3 or more carbon atoms constituting a ring including the carbon atom at position 13 of indeno-fused naphthopyran,
R¹ and R⁴ to R⁸ each independently represent a hydrogen atom or a substituent,
R² represents an electron-donating group (excluding a sulfur-containing electron-donating group),
R³ represents a hydrogen atom or an electron-donating group (excluding a sulfur-containing electron-donating group), and
B and B' each independently represent a substituent.

### [Advantageous Effects of Invention]

The compound represented by General Formula 1 can exhibit high transparency in the visible range when uncolored and a fast fading rate after coloring. Using the compound represented by General Formula 1, it is possible to provide a photochromic article that has high transparency in the visible range when uncolored and exhibits a fast fading rate after coloring.

### [Description of Embodiments]

As an example, the photochromic compound undergoes structural conversion into a colored component through an excited state when it receives light such as sunlight. The structure after structural conversion via light emission may be called a "colored component". On the other hand, the structure before light emission may be called a "colorless component". Here, regarding the colorless component, "colorless" is not limited to being completely colorless, and includes a case in which the color is lighter than that of the colored component. The structure of General Formula 1 is a structure of the colorless component.

In the present invention and this specification, the term "photochromic article" refers to an article containing a photochromic compound. A photochromic article according to one aspect of the present invention contains at least, as a photochromic compound, one or more photochromic compounds represented by General Formula 1. The photochromic compound can be incorporated into a substrate of a photochromic article and/or can be incorporated into a photochromic layer in a photochromic article having a substrate and a photochromic layer. The term "photochromic layer" refers to a layer containing a photochromic compound.

In the present invention and this specification, the term "photochromic composition" refers to a composition containing a photochromic compound. A photochromic composition according to one aspect of the present invention contains at least, as a photochromic compound, one or more photochromic compounds represented by General Formula 1, and can be used to produce a photochromic article according to one aspect of the present invention.

In the present invention and this specification, substituents in various general formulae of which details will be described below, and substituents when respective groups to be described below have substituents may be each independently,
a substituent R^{m} selected from the group consisting of a hydroxy group, linear or branched alkyl groups having 1 to 18 carbon atoms such as a methyl group, ethyl group, propyl group, butyl group, pentyl group, and hexyl group, monocyclic or polycyclic and aliphatic alkyl groups such as a bicyclic ring having 5 to 18 carbon atoms such as a cyclopentyl group and cyclohexyl group, linear or branched alkoxy groups having 1 to 24 constituent atoms such as a methoxy group, ethoxy group, and butoxy group, linear or branched perfluoroalkyl groups having 1 to 18 carbon atoms such as non-aromatic cyclic substituents having 1 to 24 constituent atoms and a trifluoromethyl group, linear or branched perfluoroalkoxy groups such as a trifluoromethoxy group, linear or branched alkylsulfide groups having 1 to 24 constituent atoms such as a methylsulfide group, ethylsulfide group, and butylsulfide group, aryl groups such as a phenyl group, naphthyl group, anthracenyl group, fluoranthenyl group, phenanthryl group, pyranyl group, perylenyl group, styryl group, and fluorenyl group, aryloxy groups such as a phenyloxy group, arylsulfide groups such as a phenylsulfide group, heteroaryl groups such as a pyridyl group, furanyl group, thienyl group, pyrrolyl group, benzofuranyl group, benzothiophenyl group, indolyl group, dibenzofuranyl group, dibenzothiophenyl group, carbazolyl group, diazolyl group, triazolyl group, quinolinyl group, phenothiazinyl group, phenoxazinyl group, phenazinyl group, thianthryl group, and acridinyl group, monoalkylamino groups such as an amino group (-NH₂) and monomethylamino group, dialkylamino groups such as a dimethylamino group, monoarylamino groups such as a monophenylamino group, diarylamino groups such as a diphenylamino group, cyclic amino groups such as a piperidino group, morpholino group, thiomorpholino group, tetrahydroquinolino group, and tetrahydroisoquinolino group, an ethynyl group, a mercapto group, a silyl group, a sulfonic acid group, an alkylsulfonyl group, a formyl group, a carboxy group, a cyano group and halogen atoms such as a fluorine atom, chlorine atom, bromine atom, and iodine atom; or
a substituent in which R^{m} is additionally substituted with one or more of the same or different R^{m}'s.

As an example of the substituent in which the above R^{m} is additionally substituted with one or more of the same or different R^{m}'s, a structure in which the terminal carbon atom of an alkoxy group is additionally substituted with an alkoxy group, and the terminal carbon atom of the alkoxy group is additionally substituted with an alkoxy group may be exemplified. In addition, as another example of the substituent in which the above R^{m} is additionally substituted with one or more of the same or different R^{m}'s, a structure in which two or more positions among five substitutable positions of a phenyl group are substituted with the same or different R^{m}'s may be exemplified. However, the present invention is not limited to such examples.

In the present invention and this specification, unless otherwise specified, groups described are substituted or unsubstituted groups. In the present invention and this specification, "substituted or unsubstituted" is synonymous with "having one or more substituents or unsubstituted". Unless otherwise specified, the terms "the number of carbon atoms" and "the number of constituent atoms" refer to the numbers including the number of carbon atoms or the number of atoms of the substituent with respect to a group having a substituent.

In addition, in the present invention and this specification, substituents in various general formulae of which details will be described below, and substituents when respective groups to be described below have substituents may each independently be a solubilizing group. In the present invention and this specification, the "solubilizing group" refers to a substituent that can contribute to increasing the compatibility with any liquid or a specific liquid. Examples of solubilizing groups include alkyl groups containing a linear, branched or cyclic structure having 4 to 50 carbon atoms, linear, branched or cyclic alkoxy groups having 4 to 50 constituent atoms, linear, branched or cyclic silyl groups having 4 to 50 constituent atoms, those in which some of the above groups are substituted with a silicon atom, sulfur atom, nitrogen atom, phosphorus atom or the like, and those obtained by combining two or more of the above groups, and a substituent that can contribute to accelerating thermal motion of molecules of the compound according to inclusion of this substituent is preferable. A compound having a solubilizing group as a substituent can inhibit the distance between solute molecules from decreasing and prevent the solute from solidifying, and can lower the melting point and/or glass transition temperature of the solute and create a molecule aggregation state close to that of a liquid. Therefore, the solubilizing group can dissolve a solute or increase the solubility of the compound having this substituent in a liquid. In one aspect, the solubilizing group is preferably an n-butyl group, n-pentyl group, n-hexyl group, and n-octyl group which are a linear alkyl group, a tert-butyl group which is a branched alkyl group, or a cyclopentyl group and a cyclohexyl group which are a cyclic alkyl group.

The substituent is preferably a substituent selected from the group consisting of a methoxy group, ethoxy group, phenoxy group, methylsulfide group, ethylsulfide group, phenylsulfide group, trifluoromethyl group, phenyl group, naphthyl group, dibenzofuranyl group, dibenzothiophenyl group, carbazolyl group, phenothiazinyl group, phenoxazinyl group, phenazinyl group, acridinyl group, dimethylamino group, diphenylamino group, piperidino group, morpholino group, thiomorpholino group, cyano group and solubilizing group, and more preferably a substituent selected from the group consisting of a methoxy group, phenoxy group, methylsulfide group, phenylsulfide group, trifluoromethyl group, phenyl group, dimethylamino group, diphenylamino group, piperidino group, morpholino group, thiomorpholino group, cyano group and solubilizing group.

In the present invention and this specification, the term "electron-donating group" refers to a substituent that more readily donates electrons to the side of atoms to which it is bonded than hydrogen atoms. Electron-donating groups may be substituents that tend to donate electrons as a summation of an inductive effect, a mesomeric effect (or a resonance effect) and the like. Specific examples of electron-donating groups include a hydroxy group: -OH, thiol group: -SH, alkoxy group: -OR (R is an alkyl group), alkylsulfide group: -SR (R is an alkyl group), arylsulfide group, acetyl group: -OCOCH₃, amino group: -NH₂, alkylamide group: -NHCOCH₃, dialkylamino group: -N(R)₂ (two R's are the same or different alkyl groups), morpholino group, piperidino group, and methyl group. Examples of preferable electron-donating groups include electron-donating groups having a negative value for the substituent constant σₚ at the para-position based on Hammett's rule.

Specific examples of the substituent constant σₚ at the para-position based on Hammett's rule (source: edited by Shu Iwamura, Ryoji Noyori, Takeshi Nakai, and Isao Kitagawa, Graduate School of Organic Chemistry (Part 1) (1988)) are shown below.
- N(CH₃)₂: -0.83
- OCH₃: -0.27
- t-C₄H₉: -0.20
- CH₃: -0.17
- C₂H₅: -0.15
- C₆H₅: -0.01(-H: 0)
- F: +0.06
- Cl: +0.27
- Br: +0.23
- CO₂C₂H₅: +0.45
- CF₃: +0.54
- CN: +0.66
- SO₂CH₃: +0.72
- NO₂: +0.78

### [Photochromic Compound Represented by General Formula 1]

Hereinafter, photochromic compounds represented by General Formula 1 will be described in more detail.

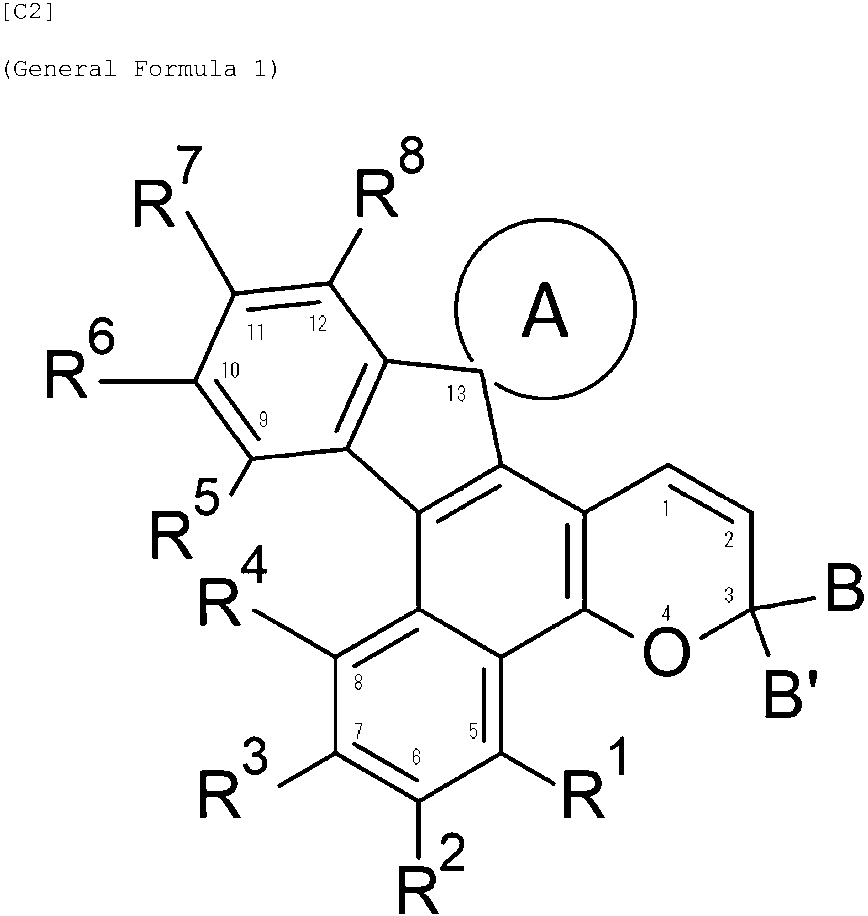

The carbon atom at position 13 of indeno-fused naphthopyran may be a spiro atom shared by the ring structure represented by A and the indeno-fused naphthopyran. That is, the ring structure represented by A may be a ring structure spiro-fused with indeno-fused naphthopyran.

In General Formula 1, A represents a ring structure having 3 or more carbon atoms constituting a ring including the carbon atom at position 13 of indeno-fused naphthopyran. The inventors speculate that having such a ring structure can contribute to the photochromic compound represented by General Formula 1 being able to exhibit a fast fading rate after coloring. However, the present invention is not limited by the speculation described in this specification.

The ring structure may be a monocyclic structure, may be a fused polycyclic structure such as a bicyclic or tricyclic structure, may be a bridged ring structure such as a bicyclic structure, and may be a spiro ring structure such as a bicyclic structure.

As the ring structure, an aliphatic ring may be exemplified. Such an aliphatic ring may be unsubstituted or may have a substituent. For substituents, the above description regarding substituents can be referred to.

As the aliphatic ring, an aliphatic ring in which the number of carbon atoms constituting the ring including the carbon atom at position 13 of indeno-fused naphthopyran is 3 or more and 20 or less may be exemplified. Specific examples thereof include monocyclic rings such as a cyclohexane ring, a cyclooctane ring, and a cycloheptane ring, bicyclo rings such as a norbornane ring and a bicyclononane ring, and tricyclo rings such as an adamantane ring. In the case of an aliphatic ring having a substituent, the "number of carbon atoms constituting a ring" includes the number of carbon atoms contained in the substituent. In addition, in the case of a ring structure having a substituent, the "number of atoms constituting the ring including the carbon atom at position 13 of indeno-fused naphthopyran" to be described below includes the number of atoms contained in the substituent.

In one aspect, when the ring structure represented by A is an aliphatic ring, the number of carbon atoms constituting such an aliphatic ring is preferably 3 or more and 6 or less, may be 3, 4, 5 or 6, and is more preferably 6. In addition, in another aspect, the number of carbon atoms constituting such an aliphatic ring is preferably 7 or more and 20 or less, may be 7, 8, 9, 10, 11, 12, 13, 14, 15, 16, 17, 18, 19 or 20, and is more preferably 7, 8 or 10.

As described above, it is desirable for a photochromic article to exhibit a fast fading rate after coloring by light emission. On the other hand, a photochromic article exhibiting a moderately fast fading rate is more desirable than a photochromic article exhibiting a very fast fading rate in some cases. In consideration of providing a photochromic article exhibiting a moderately fast fading rate, when the ring structure represented by A is an aliphatic ring, the number of carbon atoms constituting such an aliphatic ring is preferably 3 or more and 6 or less and more preferably 6.

In addition, examples of ring structures represented by A include
a fused polycycle in which one or more ring structures selected from the group consisting of an aromatic ring and an aromatic heterocycle are fused to an aliphatic ring in which the number of carbon atoms constituting the ring including the carbon atom at position 13 of indeno-fused naphthopyran is 3 or more and 20 or less;
a heterocycle in which the number of atoms constituting the ring including the carbon atom at position 13 of indeno-fused naphthopyran is 3 or more and 20 or less; and
a fused polycycle in which one or more ring structures selected from the group consisting of an aromatic ring and an aromatic heterocycle are fused to the heterocycle.

Specific examples of fused polycycles in which one or more ring structures selected from the group consisting of an aromatic ring and an aromatic heterocycle are fused to an aliphatic ring in which the number of carbon atoms constituting the ring including the carbon atom at position 13 of indeno-fused naphthopyran is 3 or more and 20 or less include a fluorene ring.

Specific examples of heterocycles in which the number of atoms constituting the ring including the carbon atom at position 13 of indeno-fused naphthopyran is 3 or more and 20 or less include a thiophene ring, a furan ring, and a pyridine ring.

Examples of fused polycycles in which one or more ring structures selected from the group consisting of an aromatic ring and an aromatic heterocycle are fused to the heterocycle include a phenylfuran ring and a biphenylthiophene ring.

Specific examples of ring structures represented by A include the following ring structures. Here, in the following, the carbon atom at position 13 is the carbon atom at position 13 of indeno-fused naphthopyran in General Formula 1. In addition, specific examples of ring structures represented by A include ring structures included in exemplary compounds listed below.

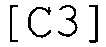

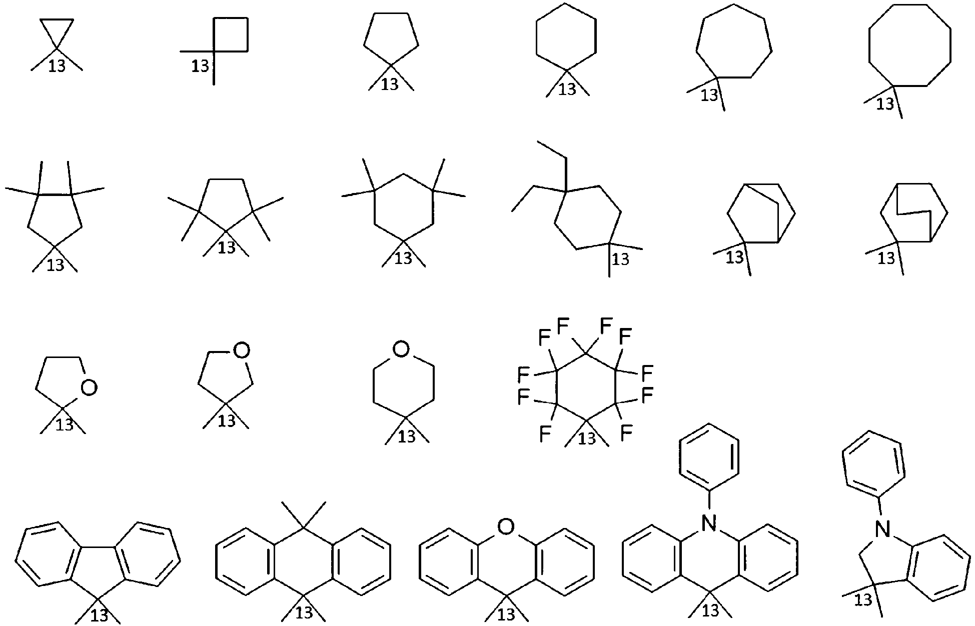

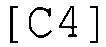

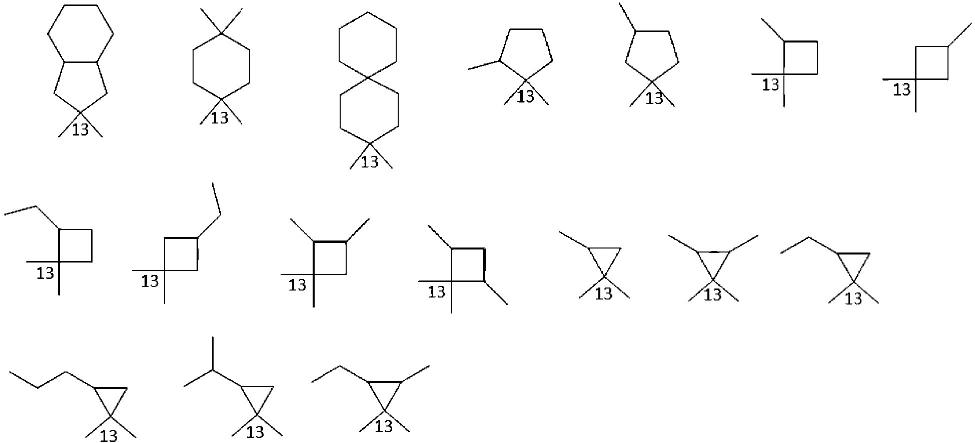

In one aspect, the ring structure represented by A is preferably the following ring structures.

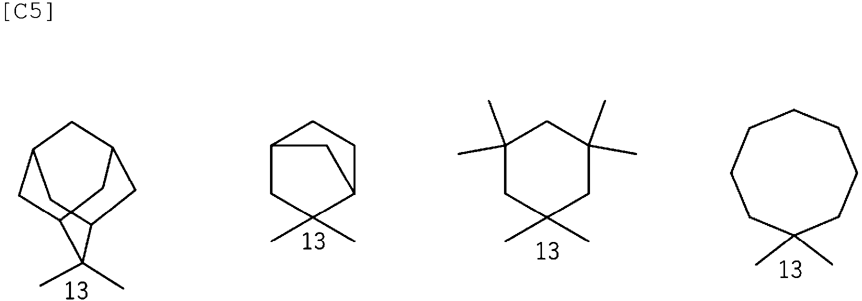

In General Formula 1, R¹ and R⁴ to R⁸ each independently represent a hydrogen atom or a substituent.

When one or more of R¹ and R⁴ to R⁸ represent a substituent, the substituent is as described above.

In one aspect, R¹ and R⁴ to R⁸ may each represent a hydrogen atom.

In another aspect, R⁷ may represent an aryl group.

In one aspect, R⁷ represents an aryl group and R¹, R⁴, R⁵, R⁶ and R⁸ may each represent a hydrogen atom.

Examples of aryl groups represented by R⁷ include aryl groups such as a phenyl group, naphthyl group, anthracenyl group, fluoranthenyl group, phenanthryl group, pyranyl group, perylenyl group, styryl group, and fluorenyl group, and a phenyl group is preferable.

When R⁷ represents an aryl group, the aryl group may be a substituted or unsubstituted aryl group. Examples of substituents that the substituted aryl group has include various substituents described above.

In one aspect, the substituted aryl group may have one or more of substituents selected from the group consisting of an alkyl group, alkoxy group and amino group.

When the substituted aryl group has an alkyl group as a substituent, examples of such alkyl groups include linear or branched alkyl groups having 1 to 18 carbon atoms such as a methyl group, ethyl group, propyl group, butyl group, pentyl group, and hexyl group, and an alkyl group selected from the group consisting of a methyl group, isopropyl group and tert-butyl group is preferable.

When the substituted aryl group has an alkoxy group as a substituent, examples of such alkoxy groups include linear or branched alkoxy group having 1 to 24 constituent atoms such as a methoxy group, ethoxy group, and butoxy group, and a methoxy group is preferable.

When the substituted aryl group has an amino group as a substituent, the amino groups may be an unsubstituted amino group (-NH₂) or a substituted amino group. The substituted amino group is preferably a dialkylamino group such as a dimethylamino group.

The substituted aryl group may be, for example, a monosubstituted aryl group or a di-substituted aryl group. In the substituted aryl group, the substitution position of the substituent is preferably a position para and/or ortho to the carbon atom at position 11 of indeno-fused naphthopyran to which the aryl group represented by R⁷ is bonded.

In General Formula 1, R² represents an electron-donating group (excluding a sulfur-containing electron-donating group), and R³ represents a hydrogen atom or an electron-donating group (excluding a sulfur-containing electron-donating group). The electron-donating group is as described above. The inventors speculate that the fact that at least R² is an electron-donating group (excluding a sulfur-containing electron-donating group) can contribute to the compound represented by General Formula 1 being able to exhibit high transparency in the visible range when no light is emitted.

Specific examples of electron-donating groups that may be represented by R² and/or R³ include an alkoxy group having 1 to 10 carbon atoms (for example, a methoxy group, and an ethoxy group), an oxoaryl group (for example, a phenoxy group), and an amino group (for example, a dimethylamino group, a piperidino group, and a morpholino group). As described above, the above groups and the following groups may be substituted or unsubstituted.

In one aspect, R² and R³ may each independently represent an electron-donating group (excluding a sulfur-containing electron-donating group).

In such an aspect, for example, the electron-donating group represented by R² may be an alkoxy group (for example, an alkoxy group having 1 to 10 carbon atoms such as a methoxy group and an ethoxy group) or a phenoxy group, and the electron-donating group represented by R³ may be an amino group. In one aspect, R² may represent an alkoxy group and R³ may represent an amino group. For example, R² may represent a methoxy group and R³ may represent an amino group. In addition, R⁷ may represent an aryl group, for example, a phenyl group.

The amino group represented by R³ may be, for example, a morpholino group or a piperidino group. For example, R² may represent a methoxy group and R³ may represent a morpholino group or a piperidino group. In addition, R⁷ may represent an aryl group, for example, a phenyl group.

For example, the electron-donating group represented by R² and the electron-donating group represented by R³ may each independently represent an alkoxy group (for example, an alkoxy group having 1 to 10 carbon atoms such as a methoxy group and an ethoxy group). In one aspect, R² and R³ may each represent a methoxy group. In addition, R⁷ may represent an aryl group, for example, a phenyl group.

In another aspect, R² may represent an electron-donating group (excluding a sulfur-containing electron-donating group) and R³ may represent a hydrogen atom. In such an aspect, for example, the electron-donating group represented by R² may be an alkoxy group (for example, an alkoxy group having 1 to 10 carbon atoms such as a methoxy group and an ethoxy group).

In General Formula 1, B and B' each independently represent a substituent.

In one aspect, B and B' may each independently be a substituted or unsubstituted phenyl group. The phenyl group having a substituent may be a 1- to 5-substituted phenyl group. In the phenyl group having two or more substituents, these substituents may be the same or different substituents, and two or more of these substituents may be bonded to form a ring structure. For substituents that the phenyl group has, the above description regarding substituents can be referred to.

In addition, in one aspect, at least one of B and B' may represent a phenyl group having a substituent at a substitution position para to the carbon atom at the position bonding to a pyran ring of indeno-fused naphthopyran. In this case, only one of B and B' may represent a phenyl group having a substituent at a substitution position para to the carbon atom at the position bonding to a pyran ring of indeno-fused naphthopyran. In addition, both B and B' may each independently represent a phenyl group having a substituent at a substitution position para to the carbon atom at the position bonding to a pyran ring of indeno-fused naphthopyran. The substituent substituted at the above substitution position para may be, for example, an electron-donating group. For electron-donating groups, the above description regarding electron-donating groups can be referred to. Specific examples of the electron-donating group substituted at the above substitution position para include an alkoxy group such as a methoxy group and an amino group such as a dimethylamino group, morpholino group, and piperidino group.

In addition, in one aspect, at least one of B and B' may be a nitrogen atom-containing phenyl group having a substituent at a substitution position para to the carbon atom at the position bonding to a pyran ring of indeno-fused naphthopyran. In this case, only one of B and B' may be a nitrogen atom-containing phenyl group having a substituent at a substitution position para to the carbon atom at the position bonding to a pyran ring of indeno-fused naphthopyran. In addition, both B and B' may each independently represent a nitrogen atom-containing phenyl group having a substituent at a substitution position para to the carbon atom at the position bonding to a pyran ring of indeno-fused naphthopyran. Examples of nitrogen atom-containing substituents include unsubstituted amino groups (-NH₂), substituted amino groups (for example, monoalkyl amino groups such as a monomethyl amino group, dialkyl amino groups such as a dimethylamino group, monoaryl amino groups such as a monophenyl amino group, and diaryl amino groups such as a diphenyl amino group), and cyclic amino groups (for example, a piperidino group, a morpholino group, a thiomorpholino group, a tetrahydroquinolino group, and a tetrahydroisoquinolino group).

Examples of compounds represented by General Formula 1 include the following compounds. Specific examples of respective parts in General Formula 1 include those included in the following exemplary compounds. However, the present invention is not limited to the following exemplary compounds.

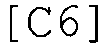

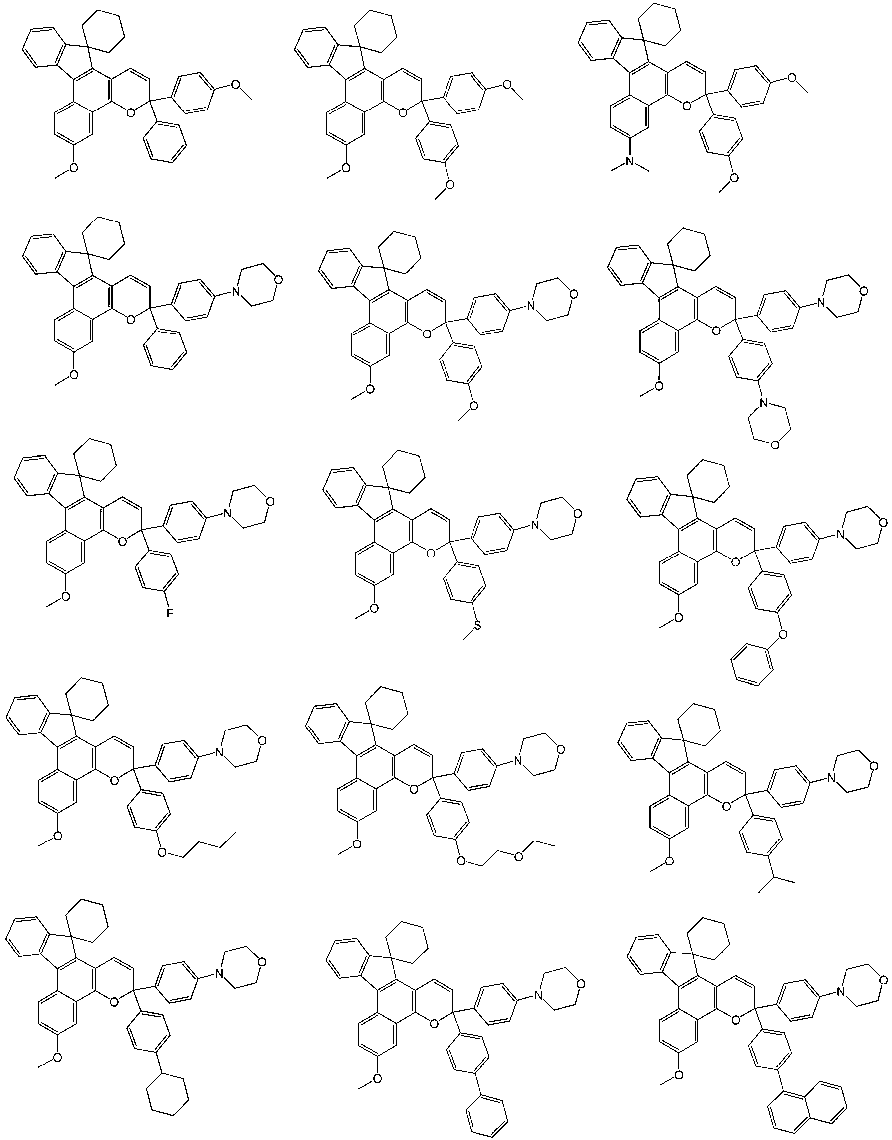

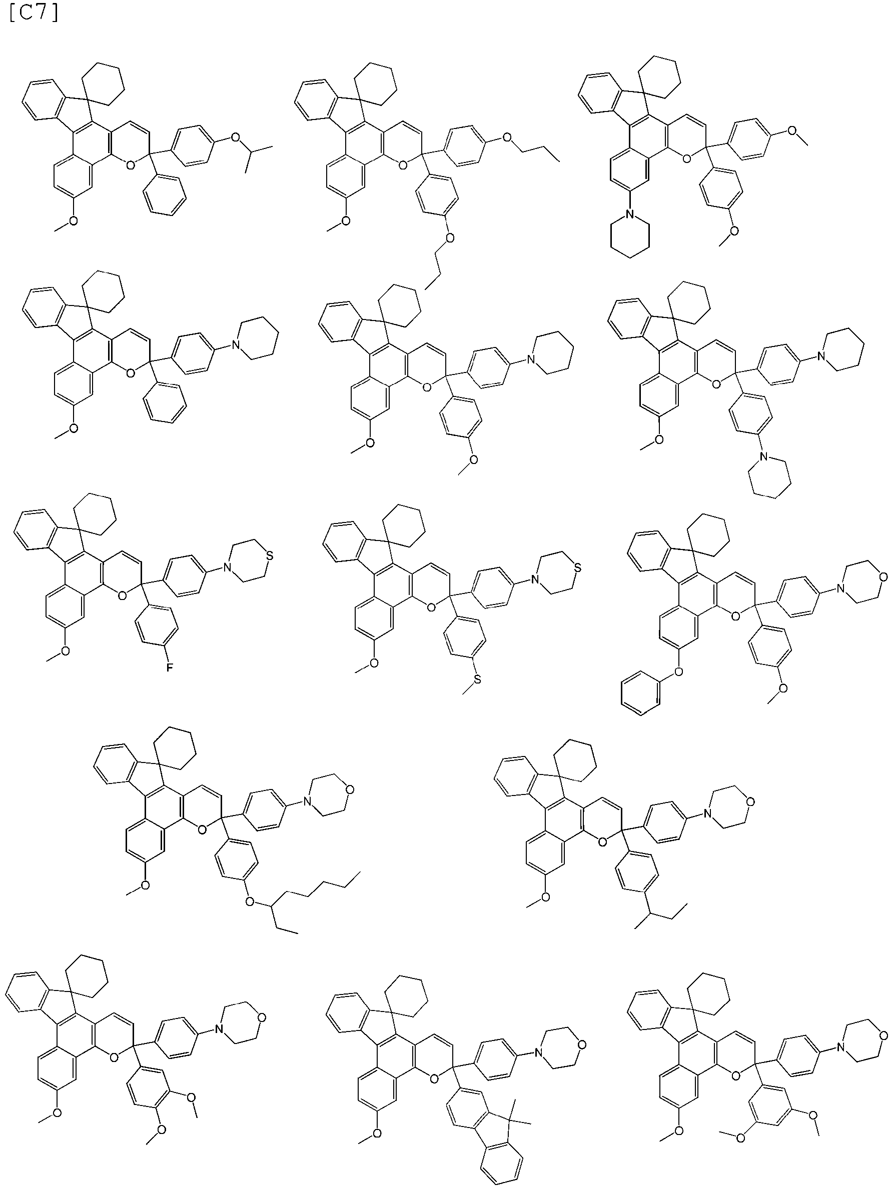

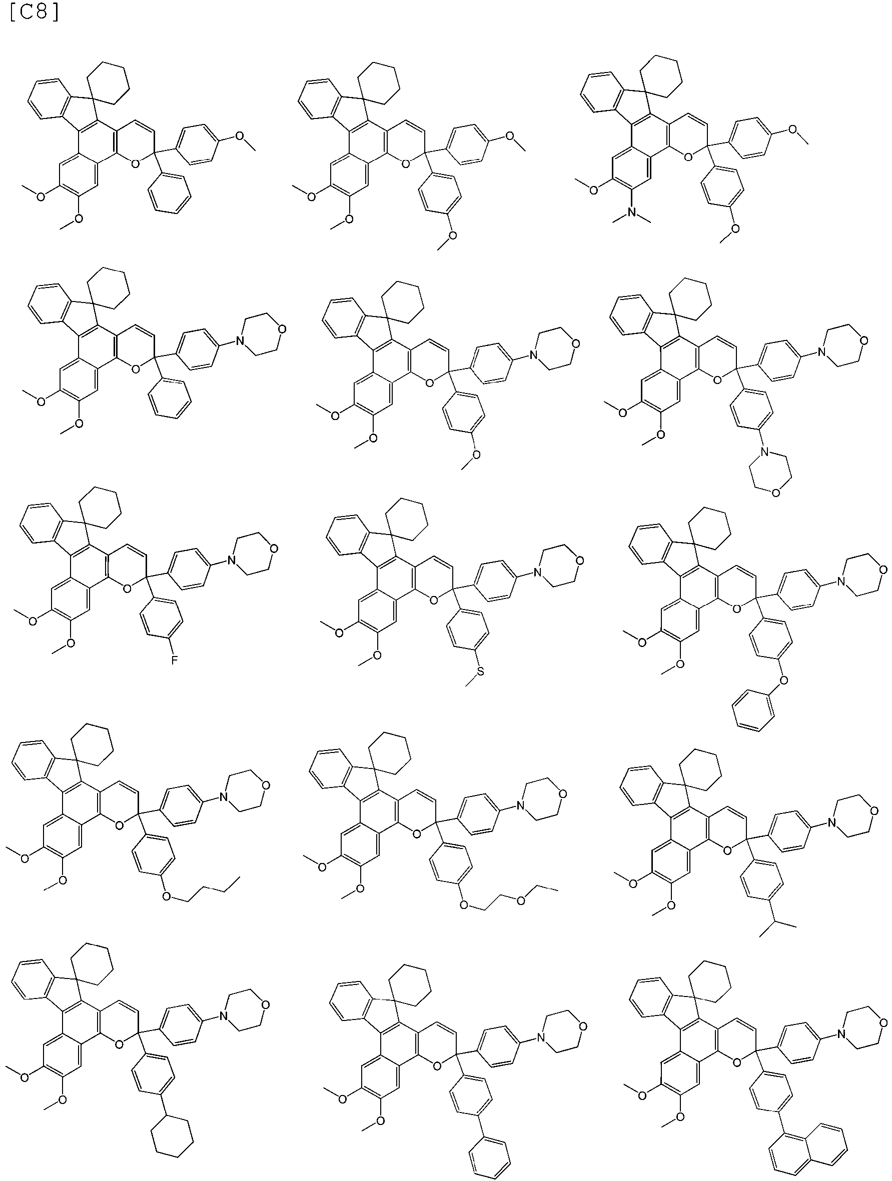

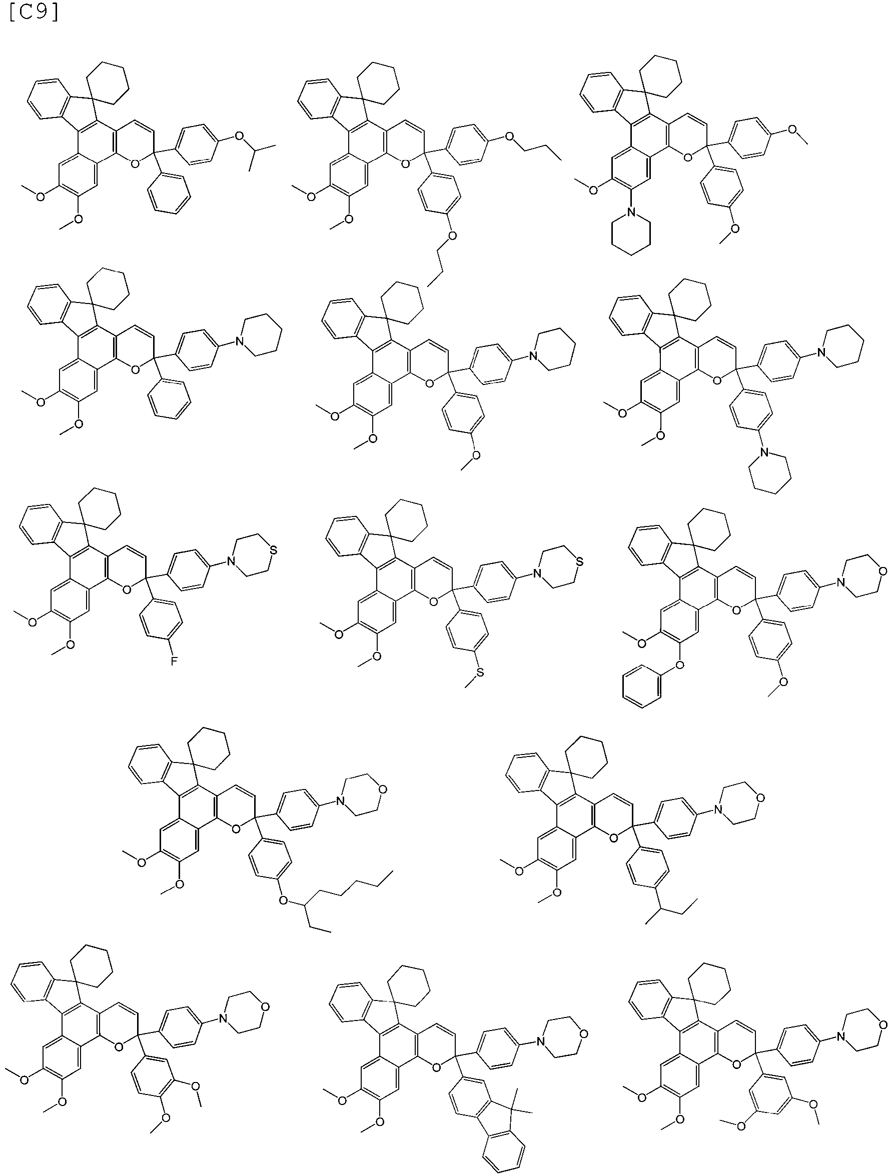

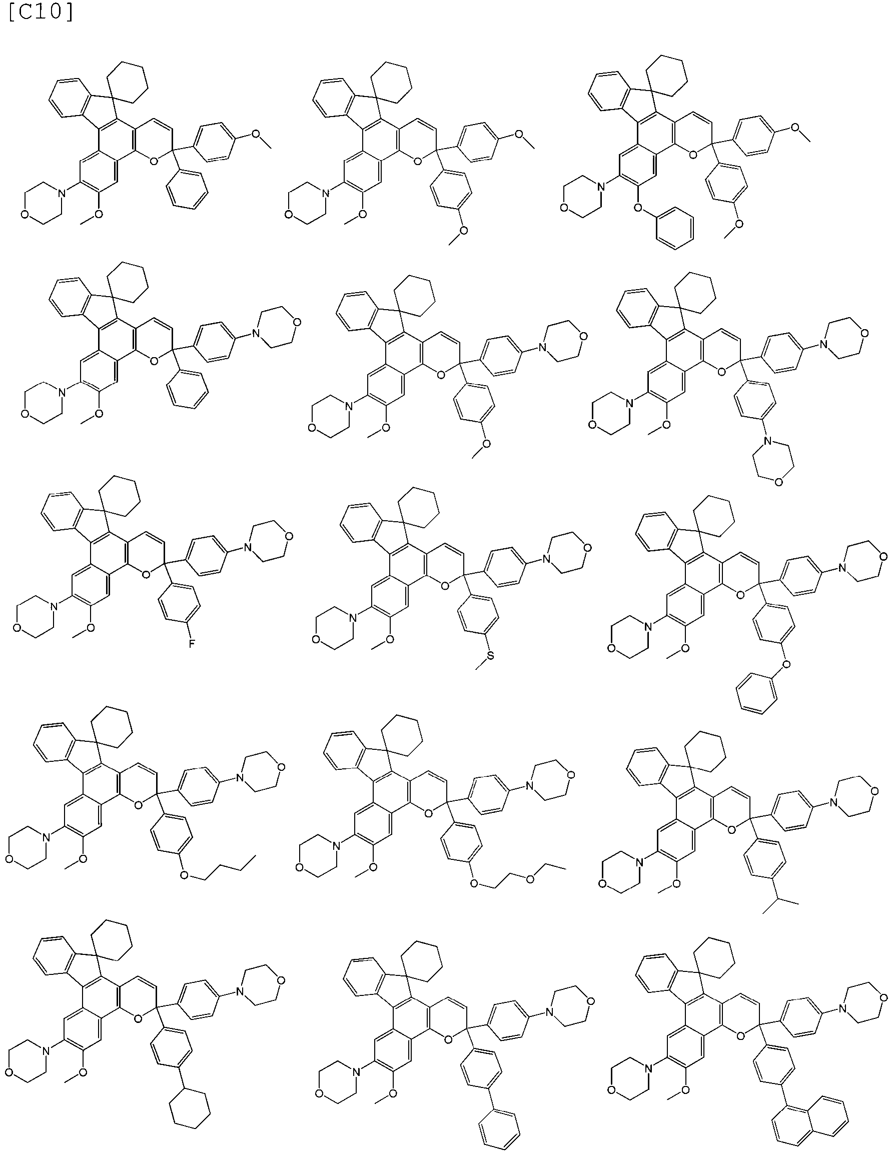

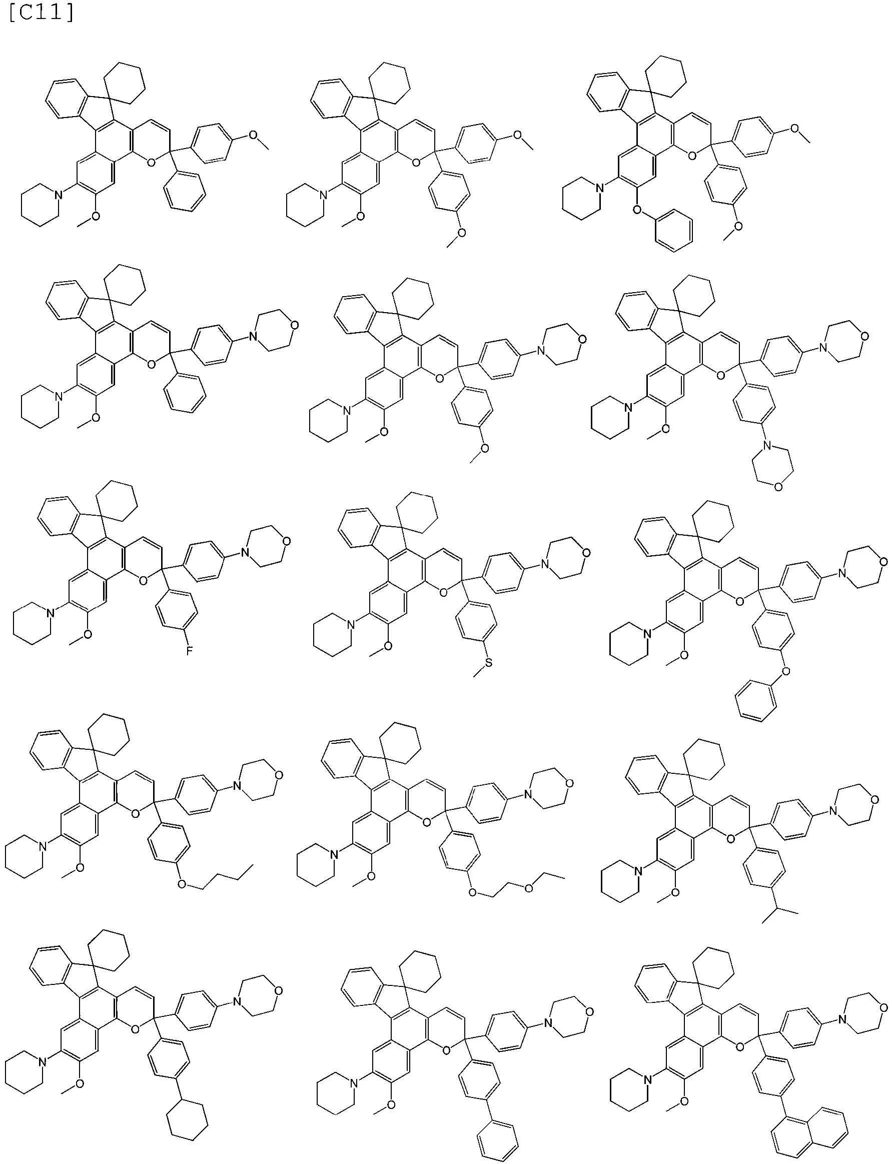

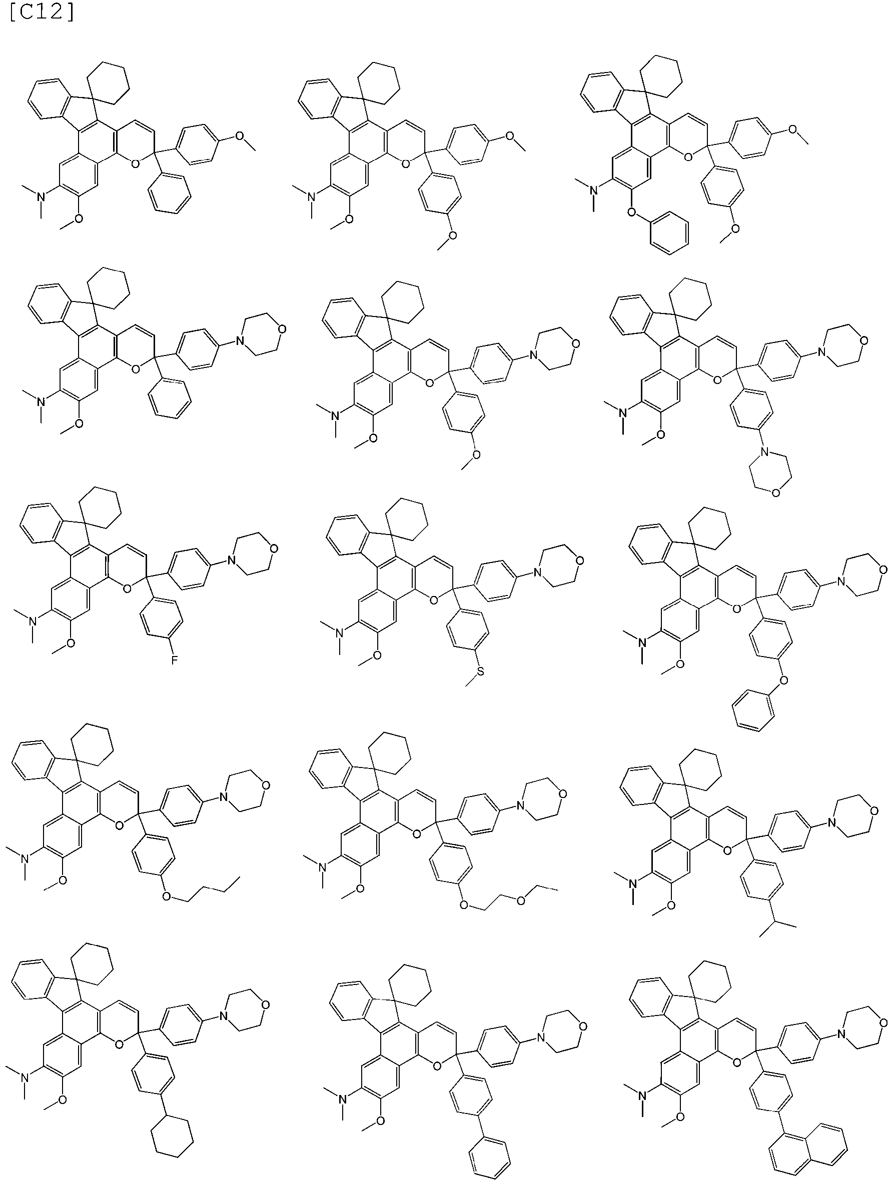

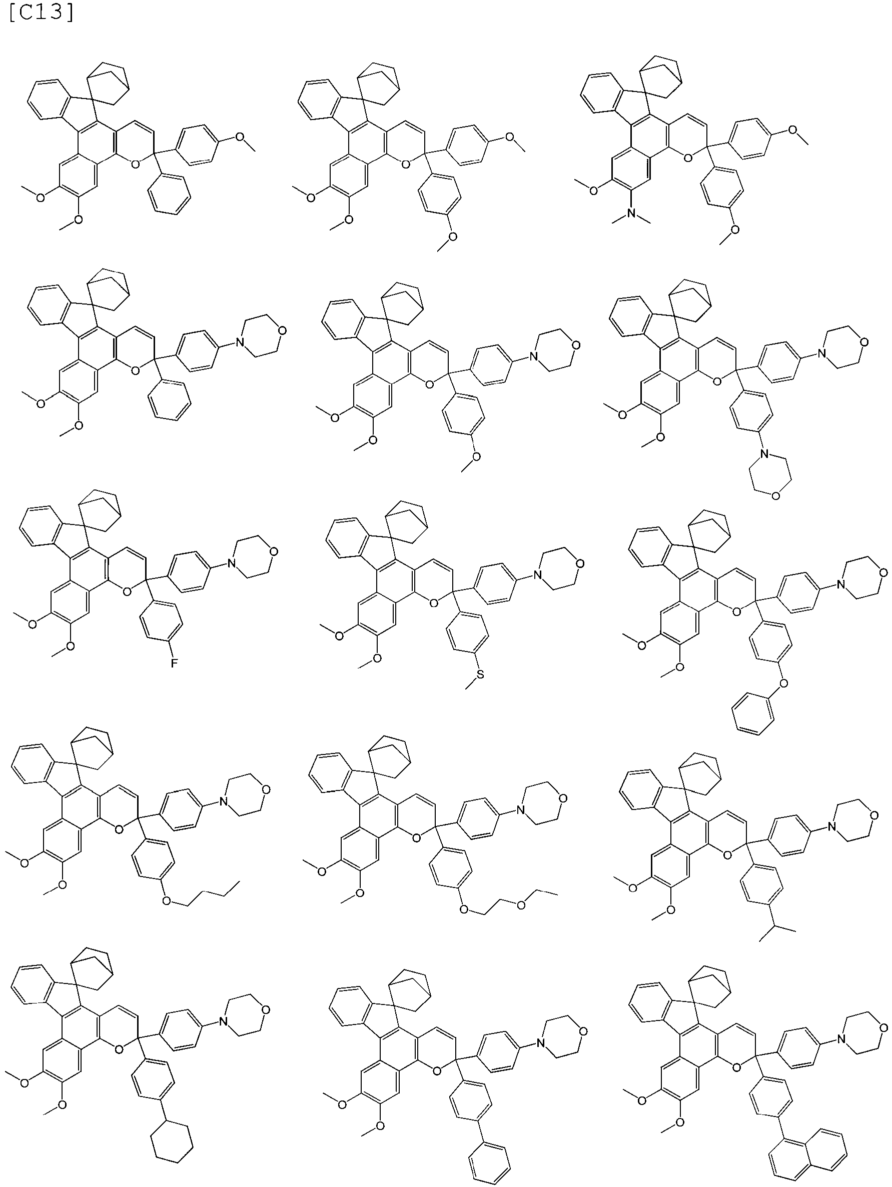

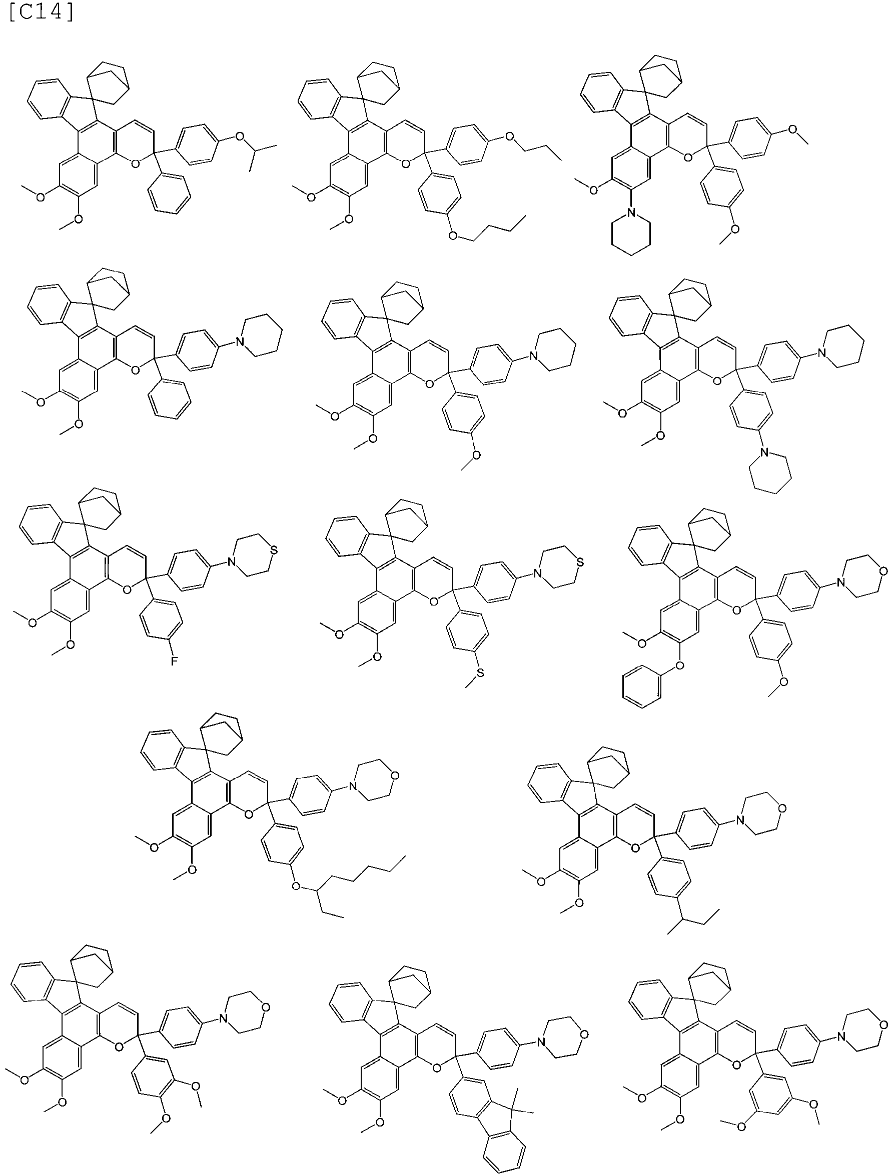

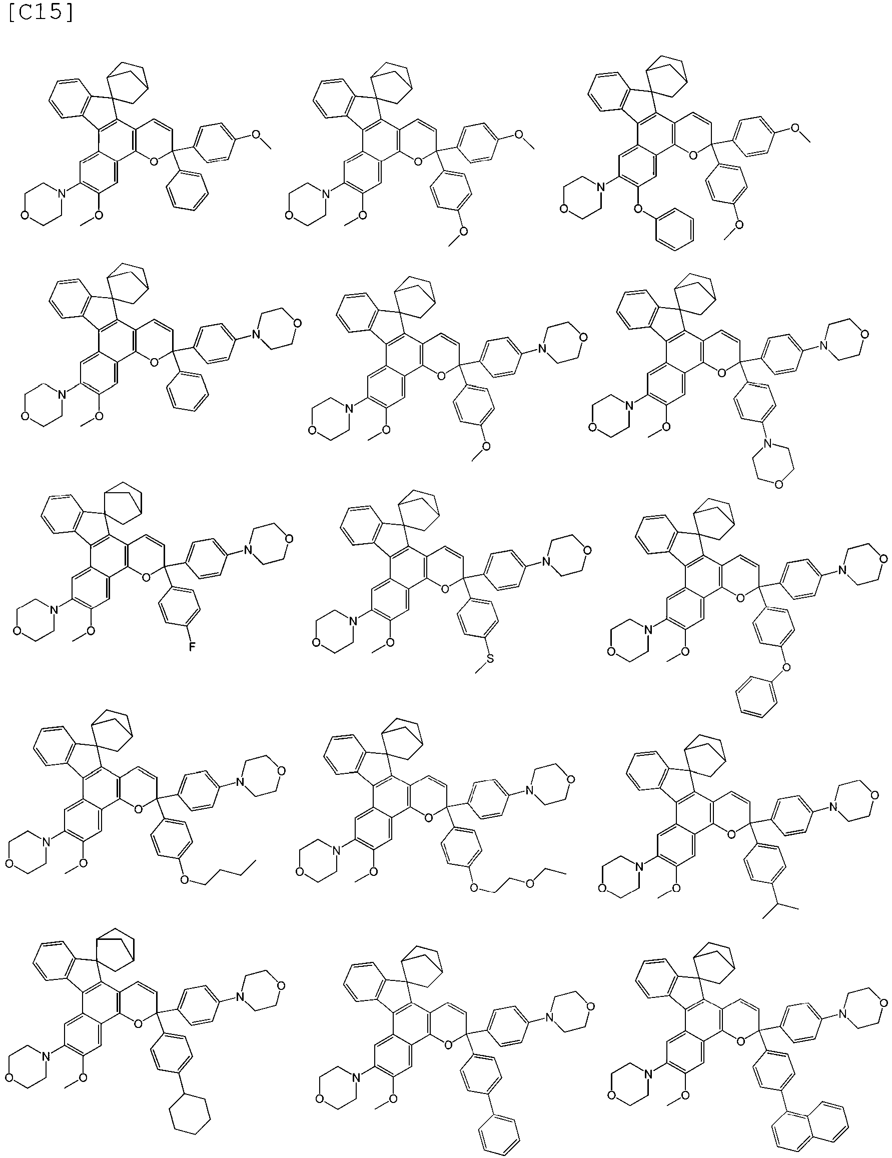

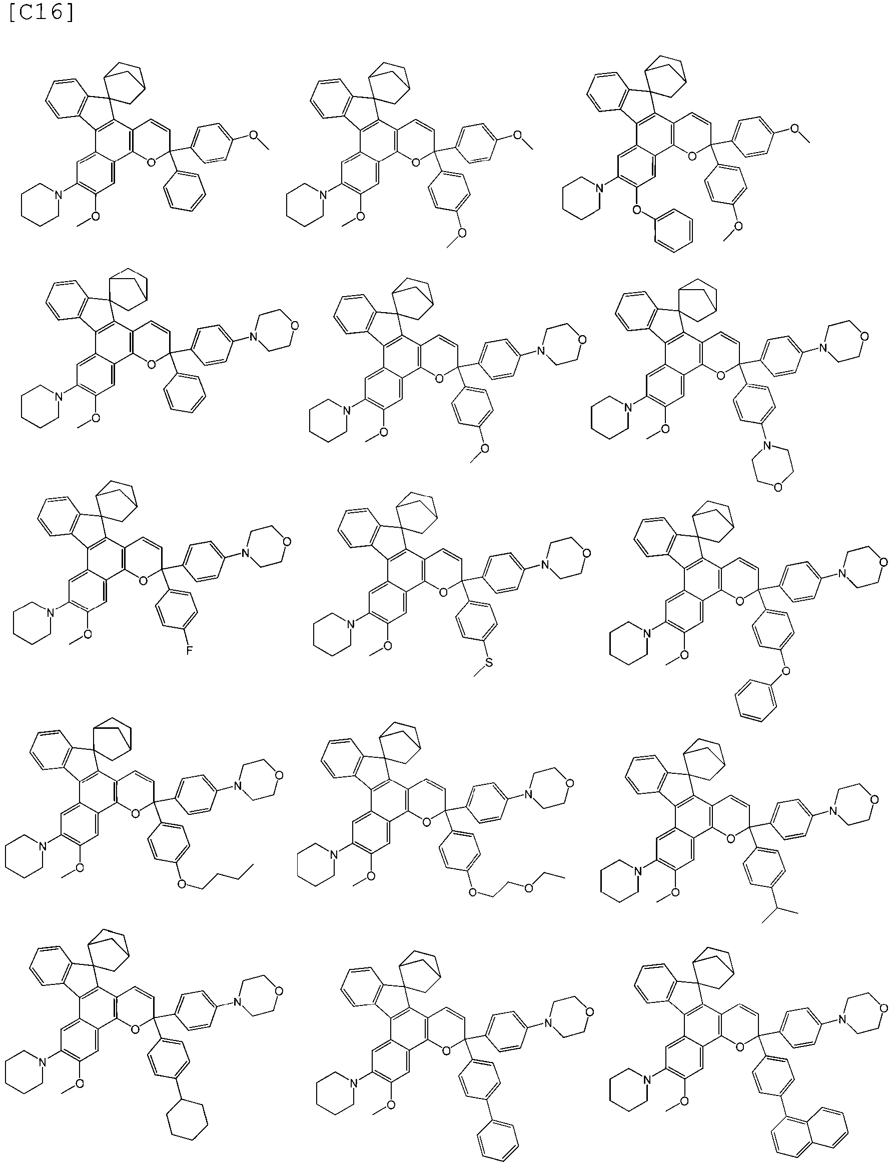

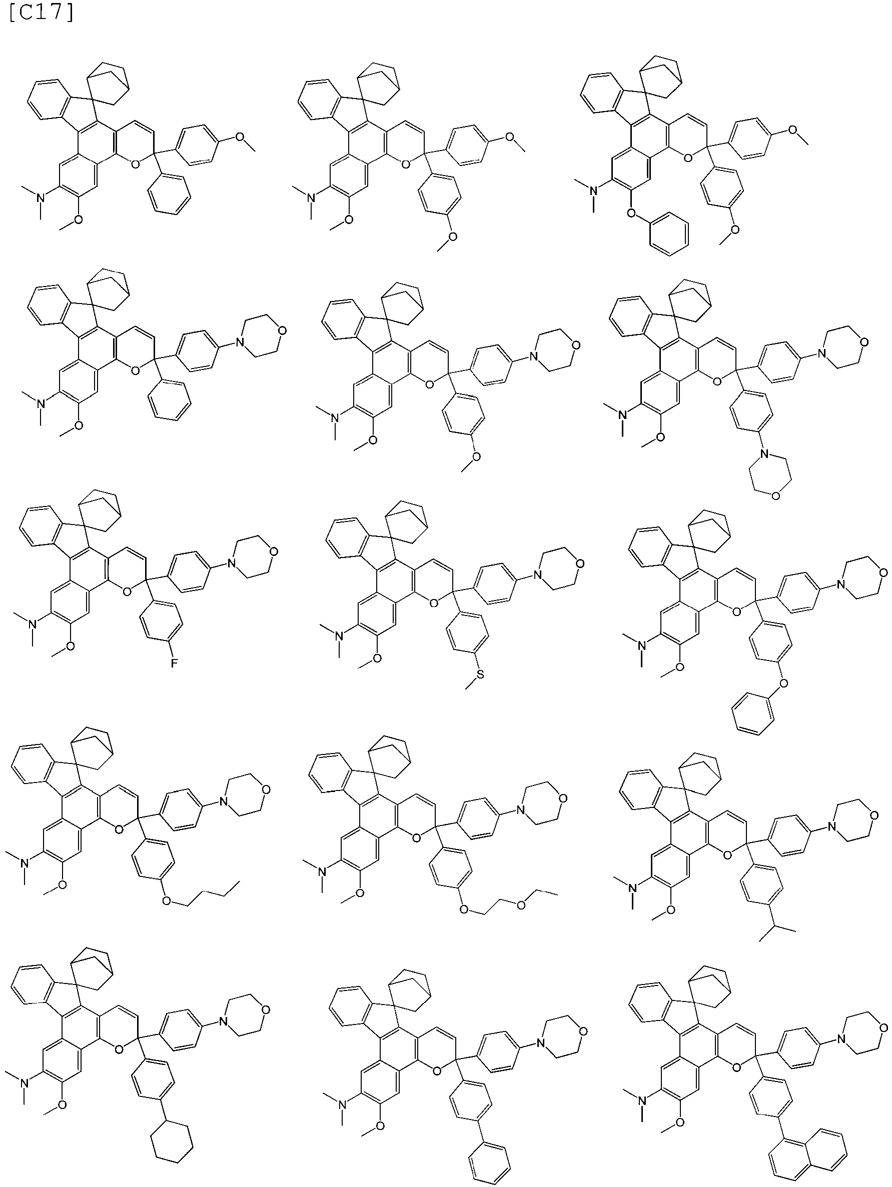

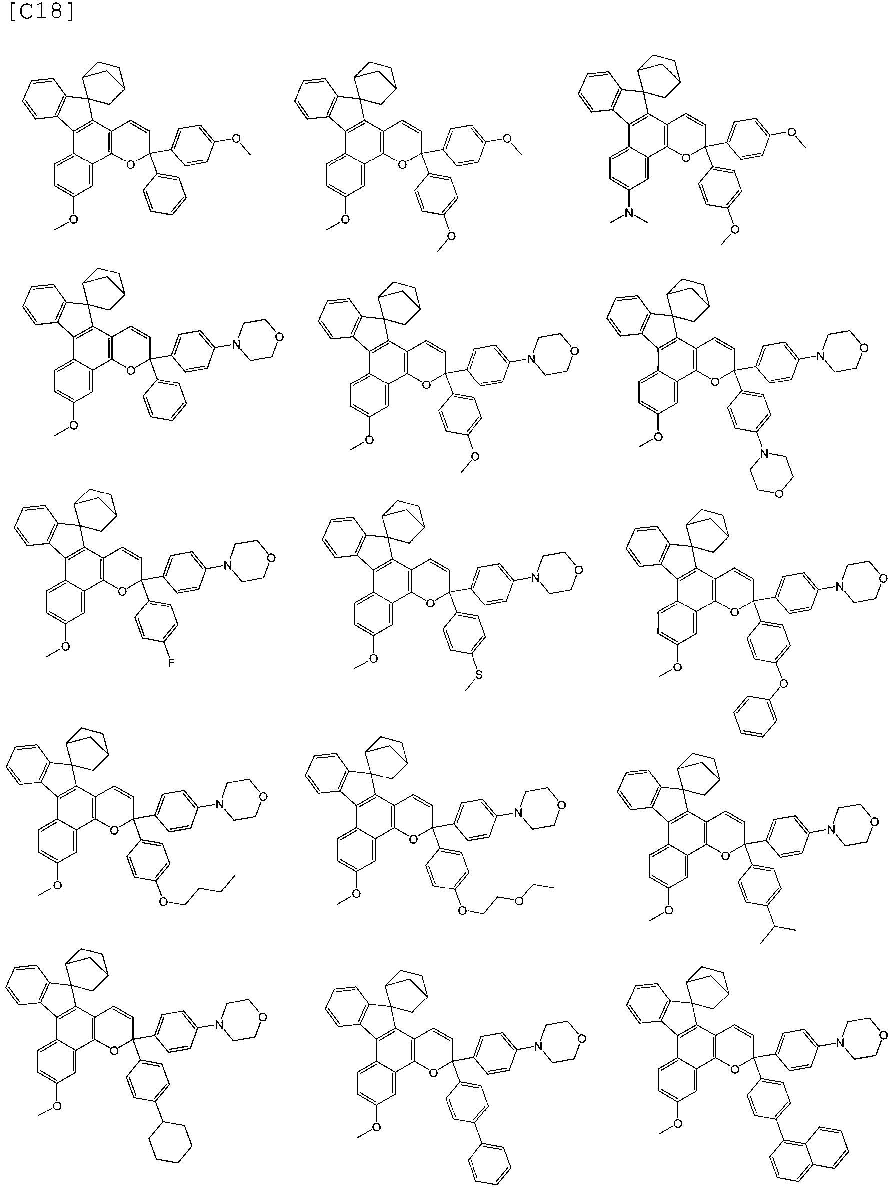

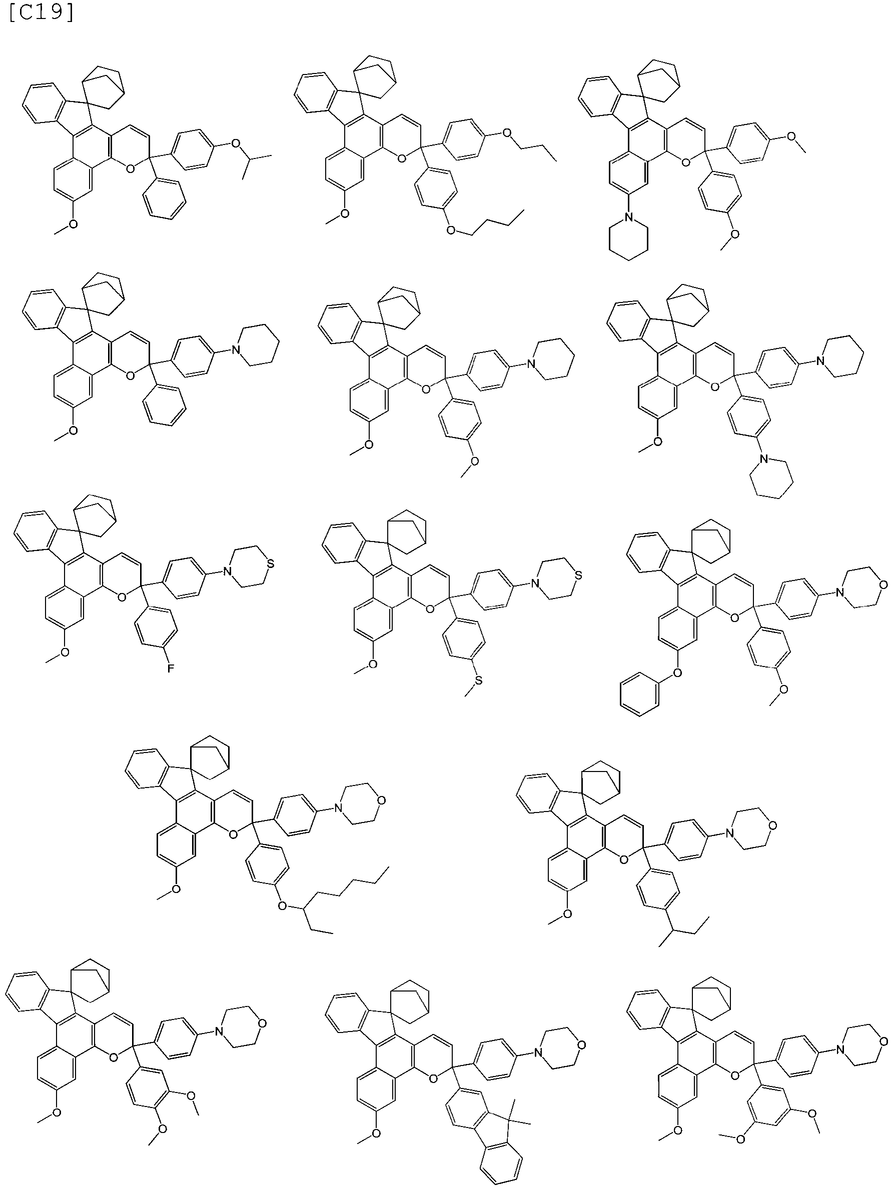

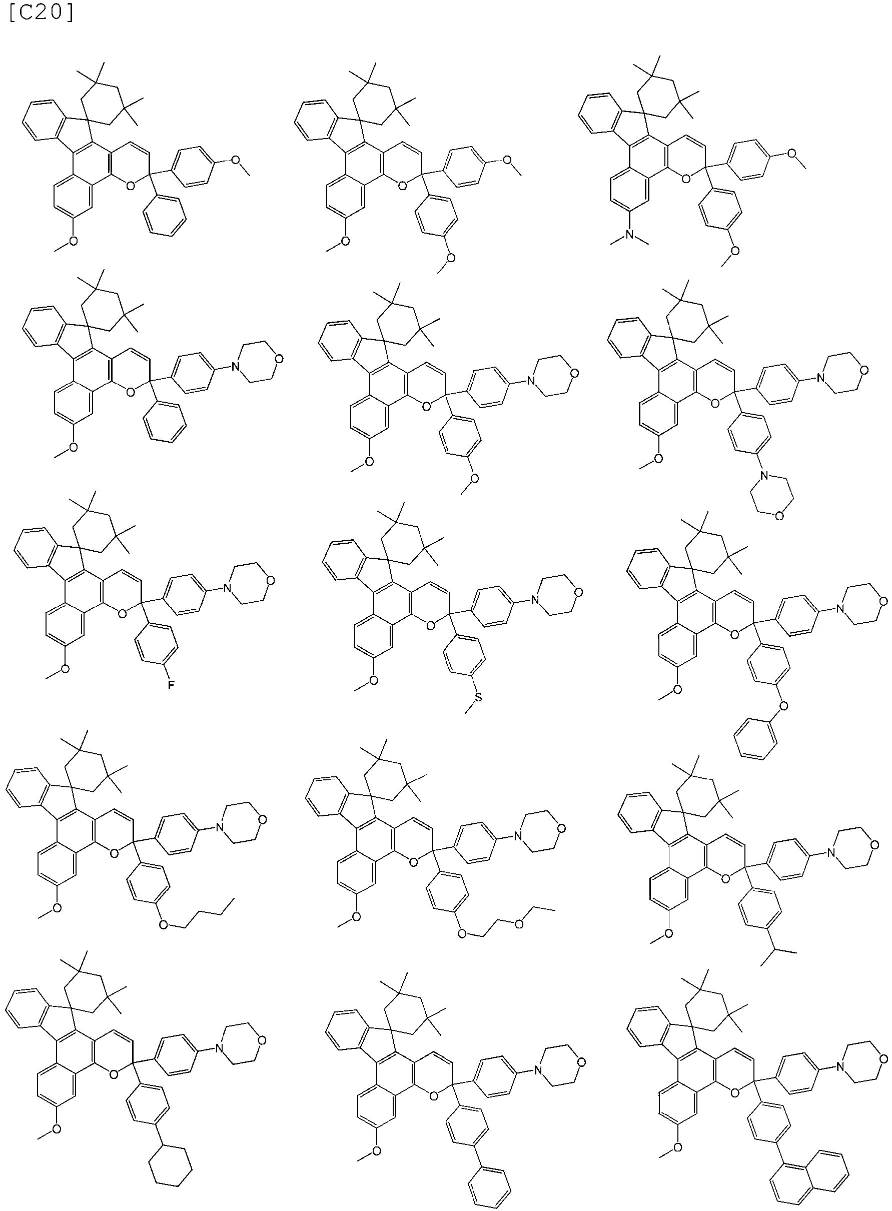

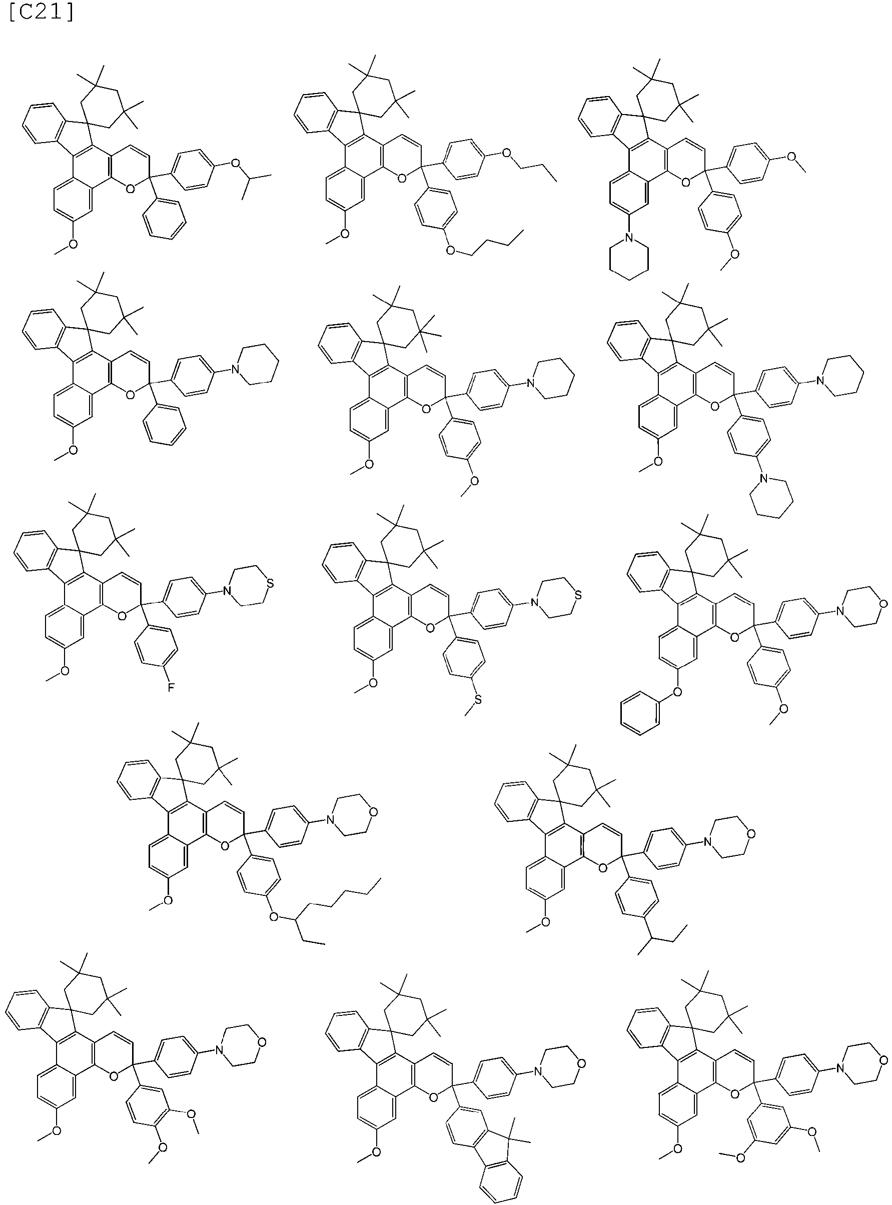

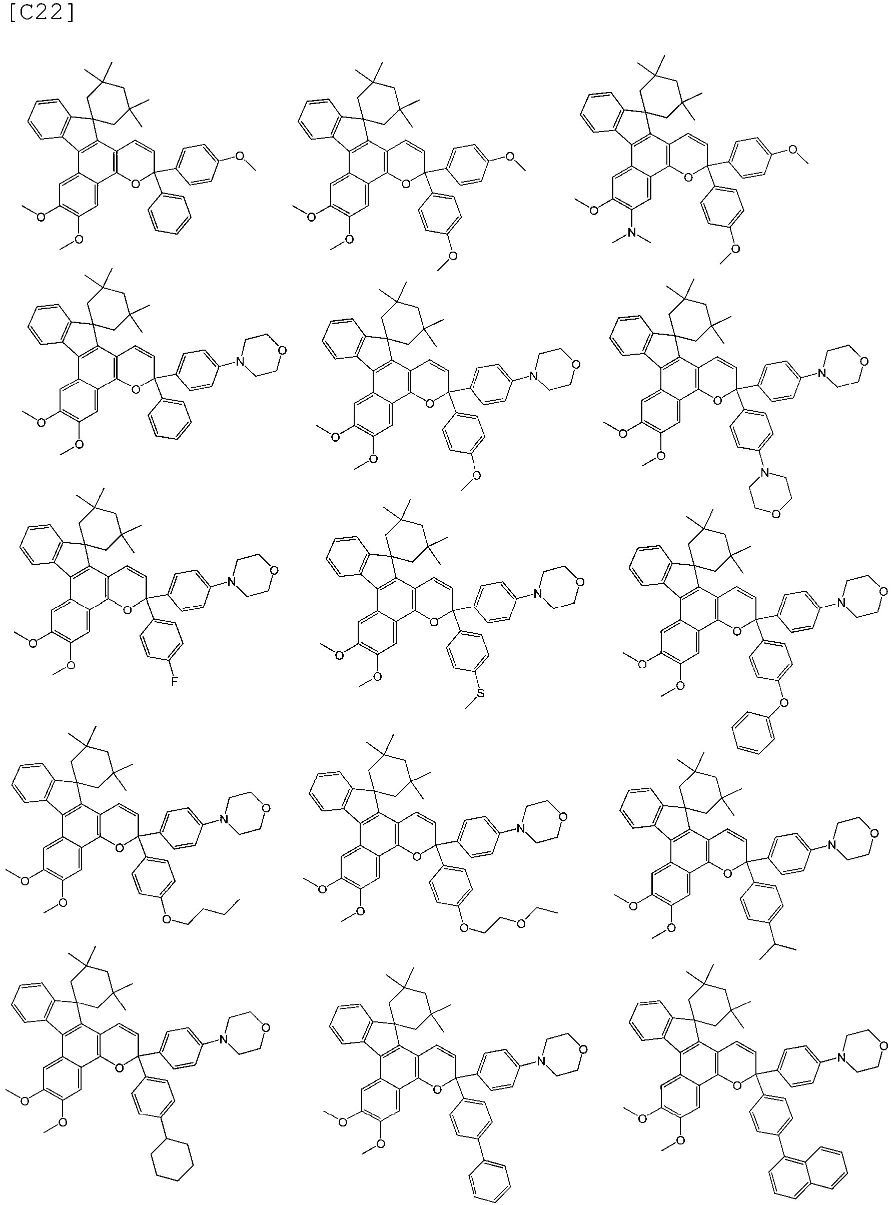

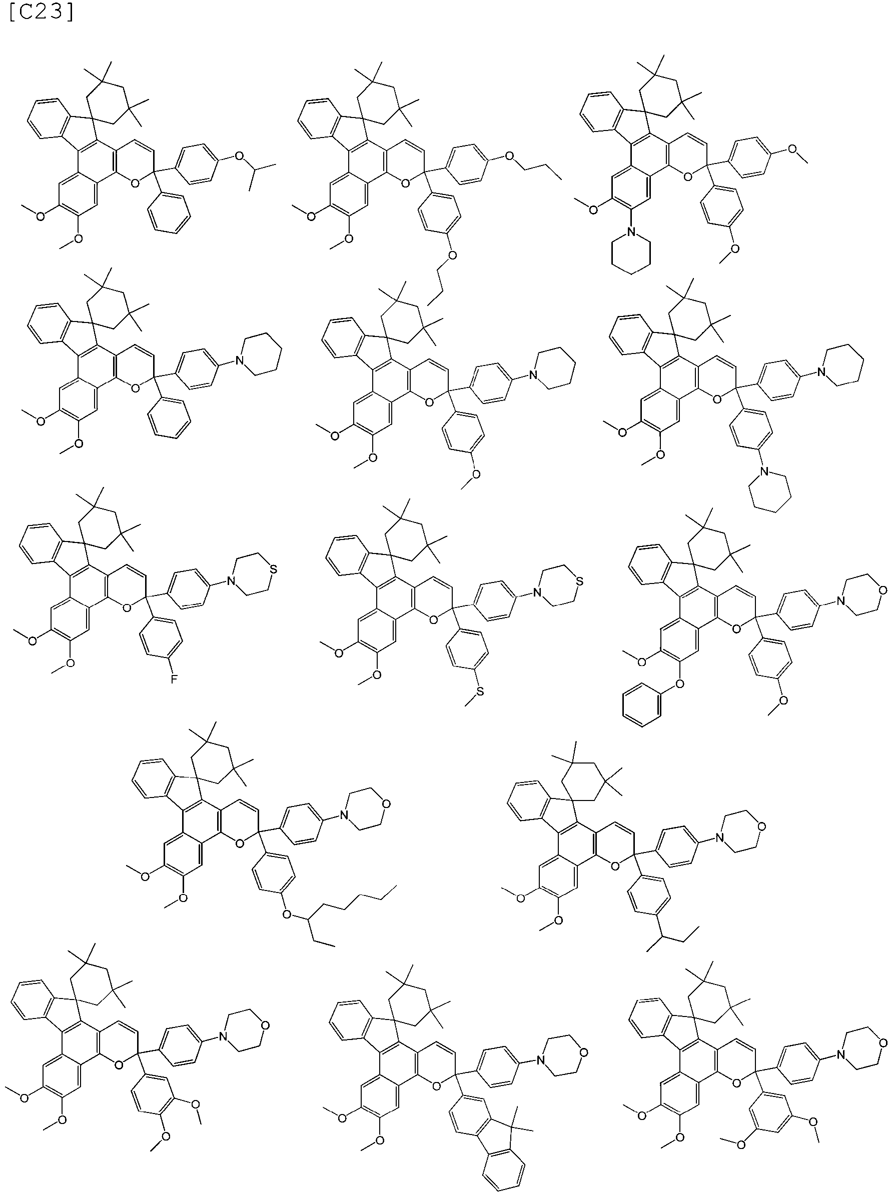

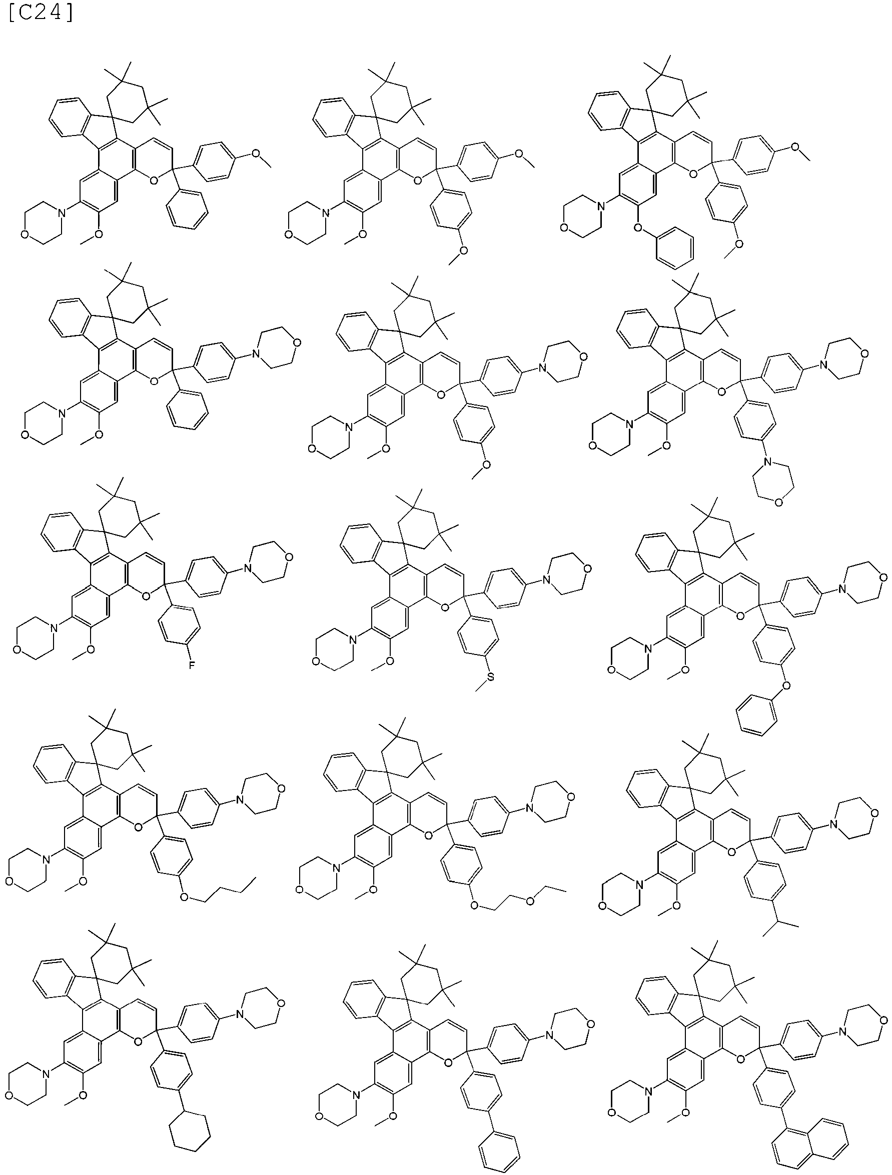

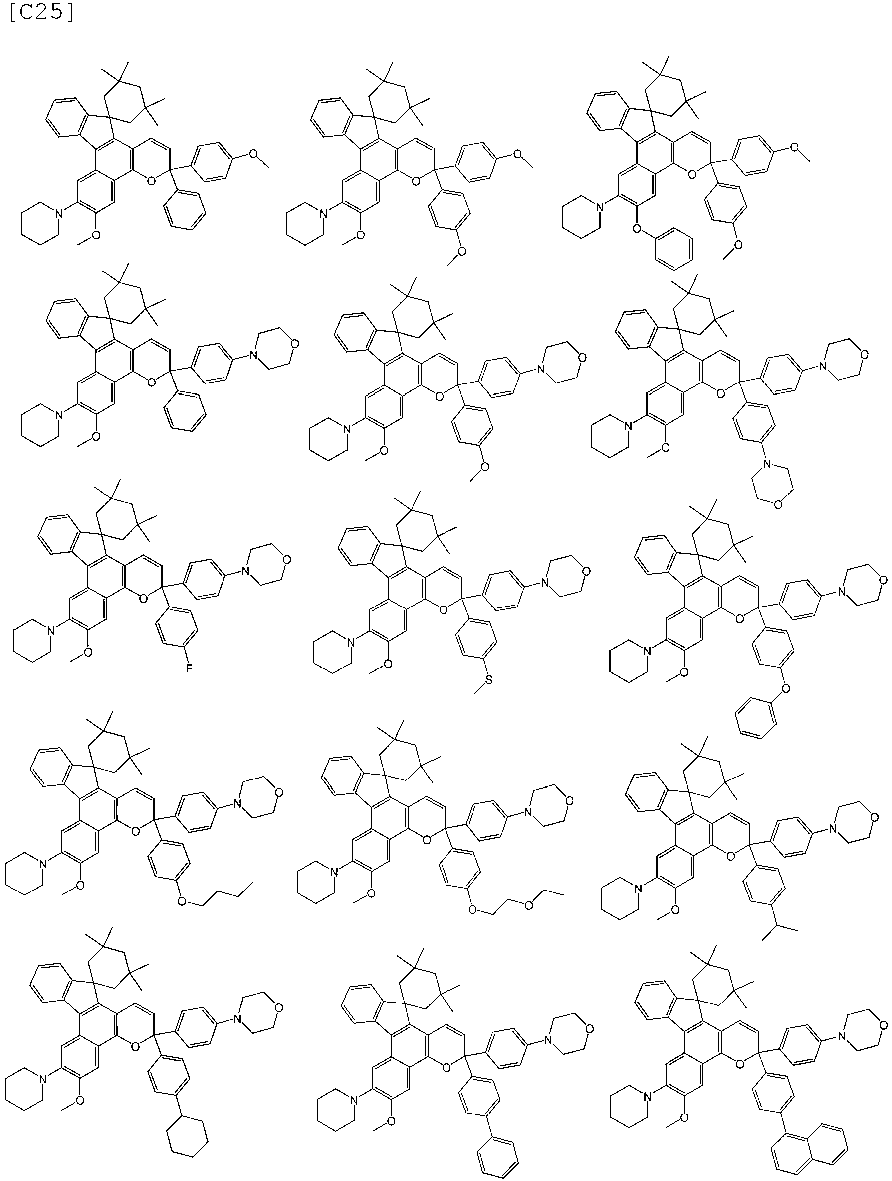

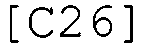

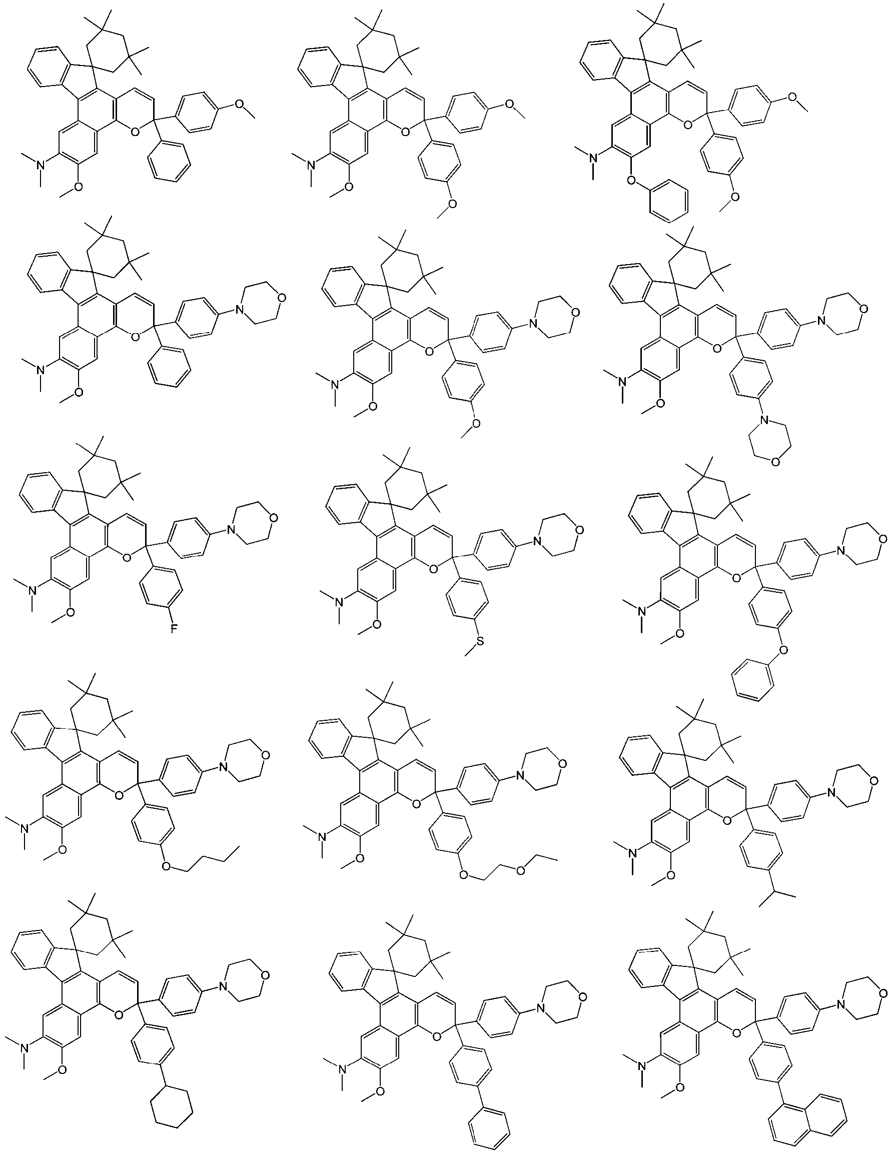

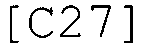

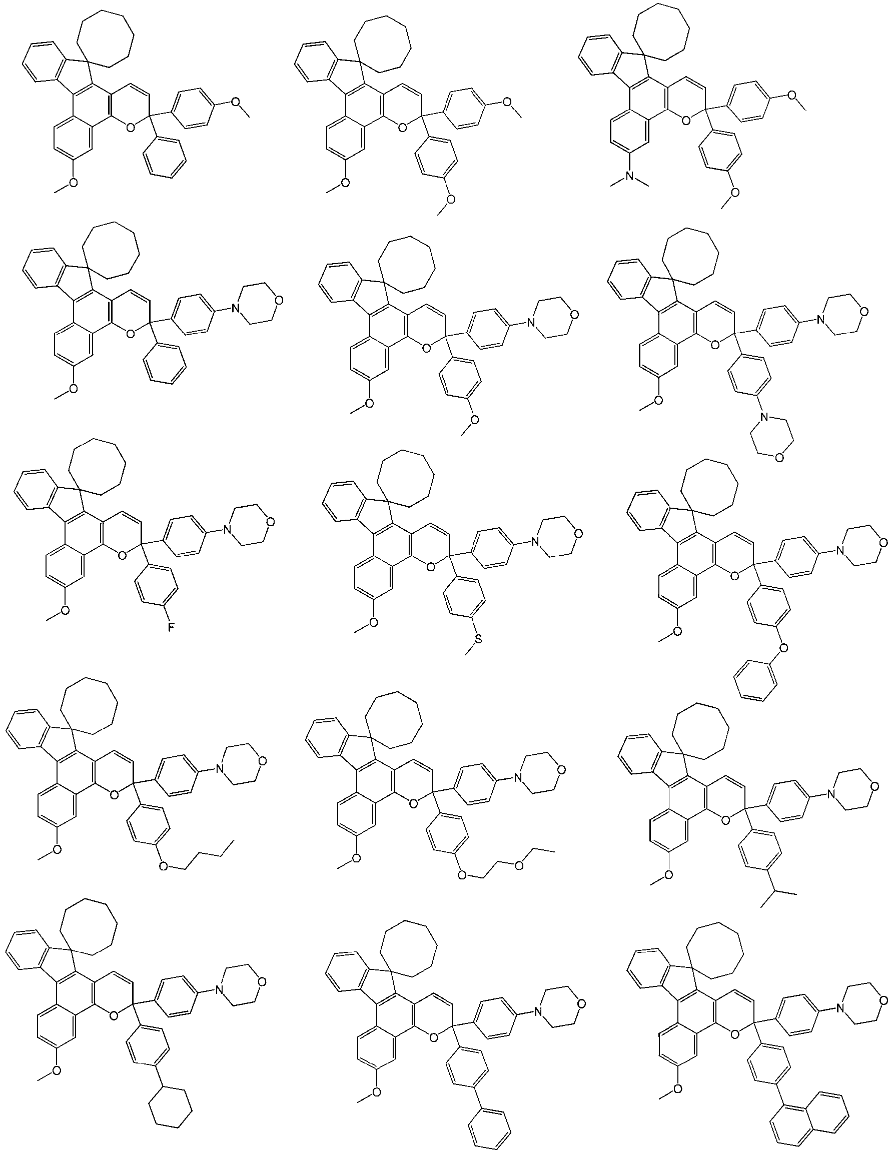

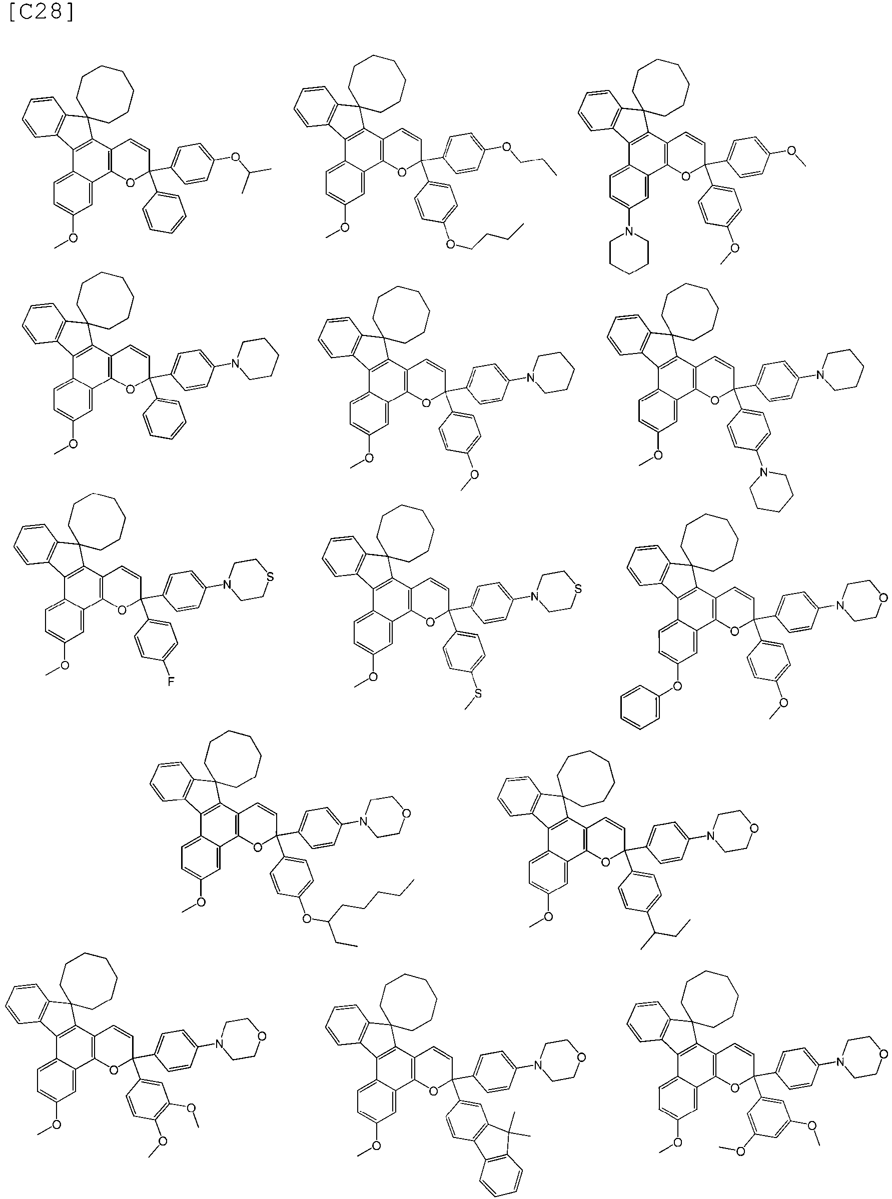

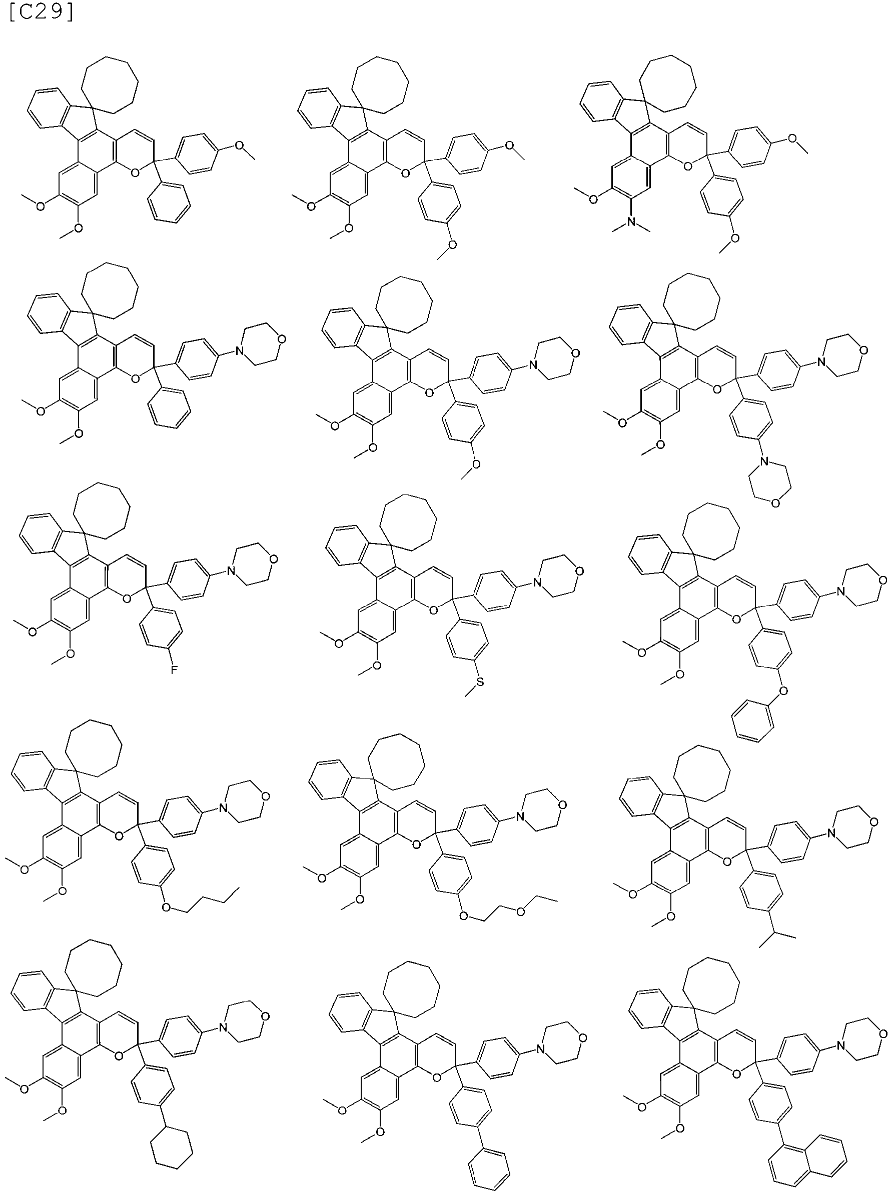

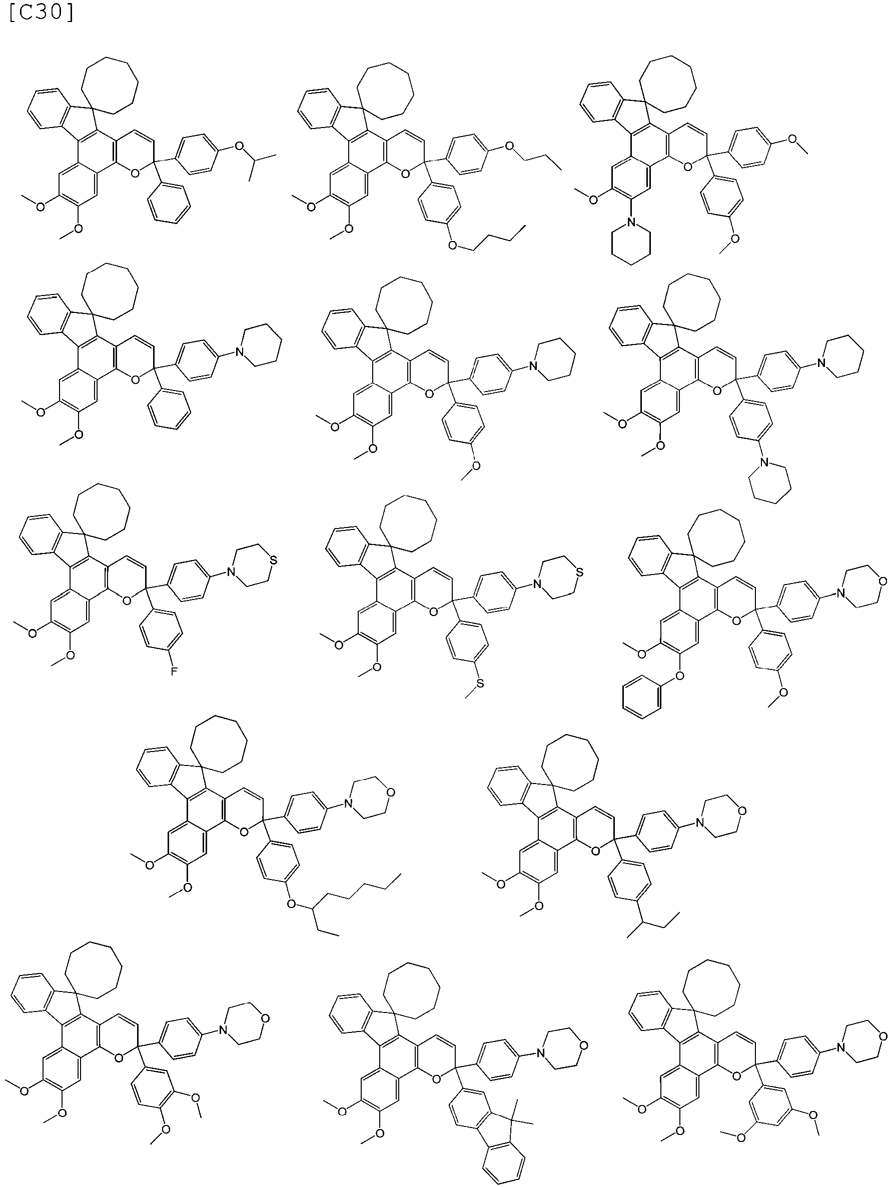

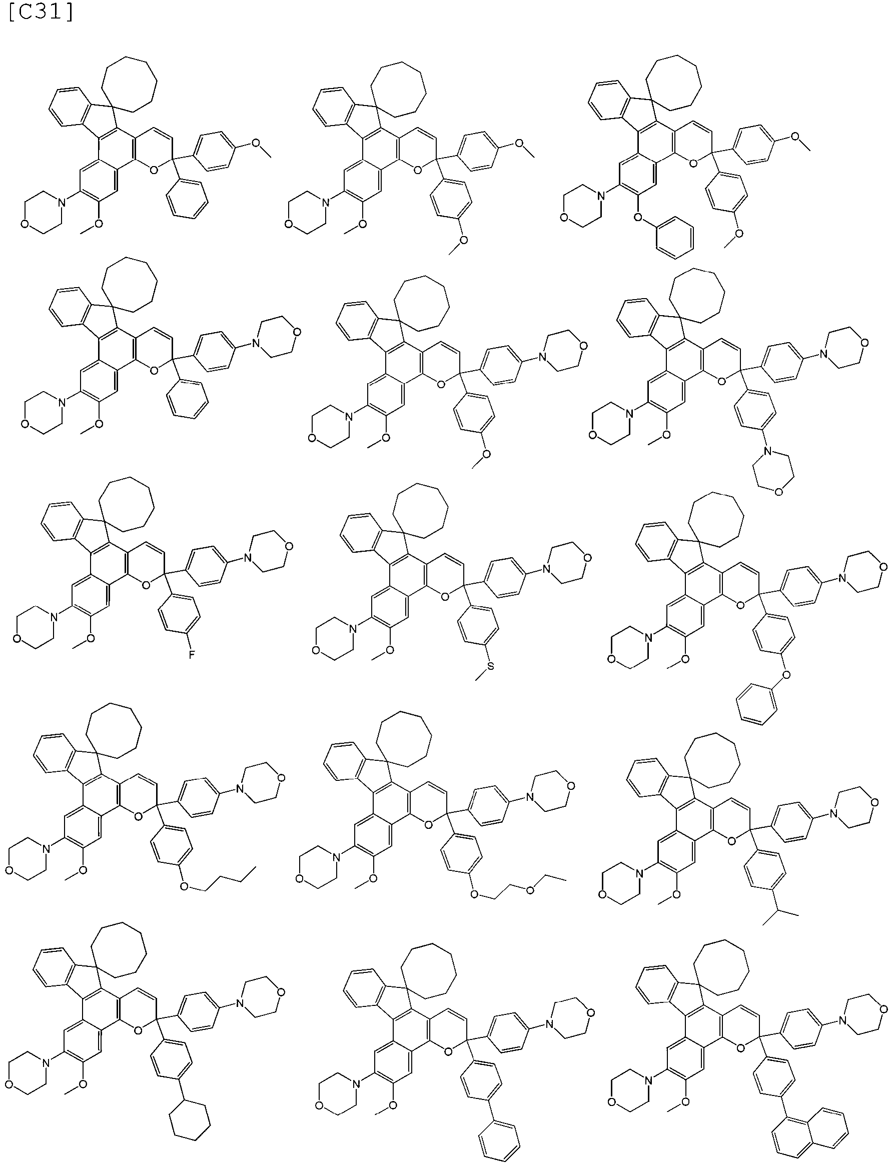

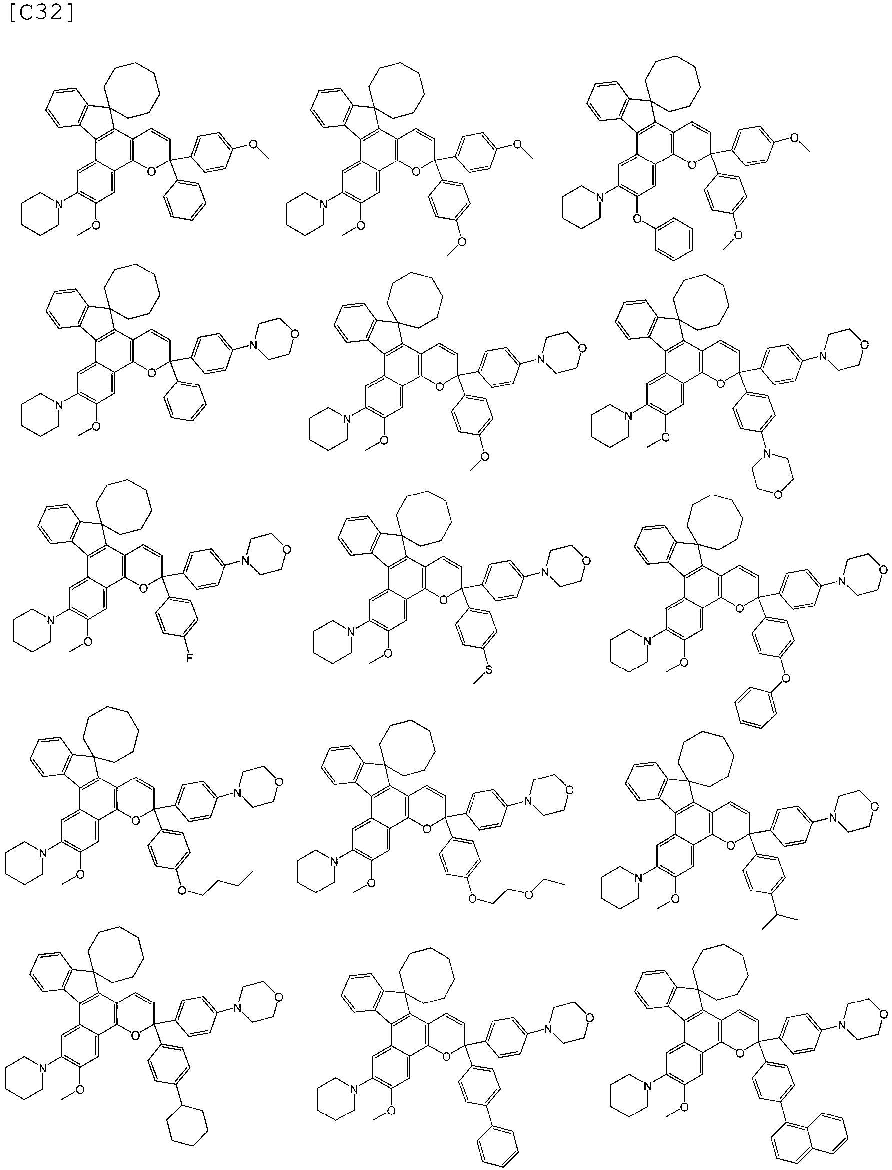

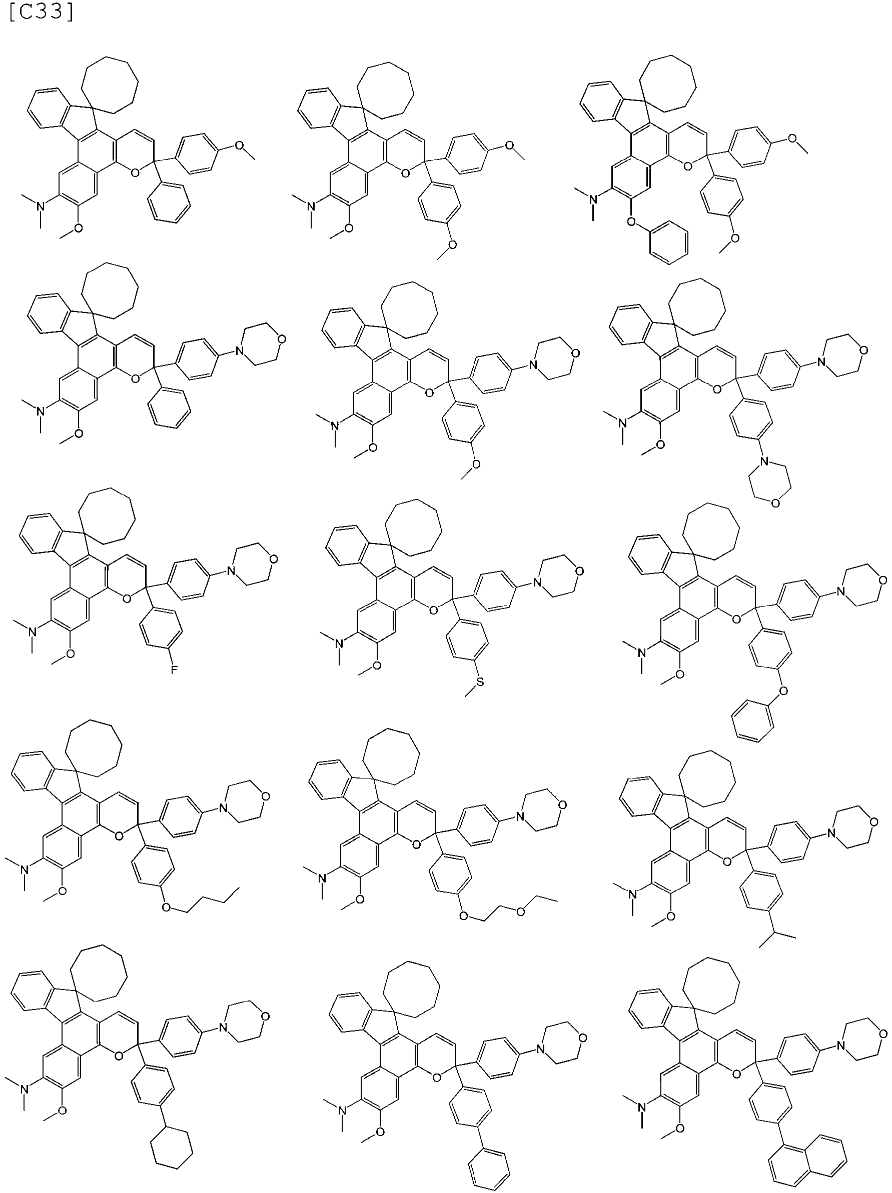

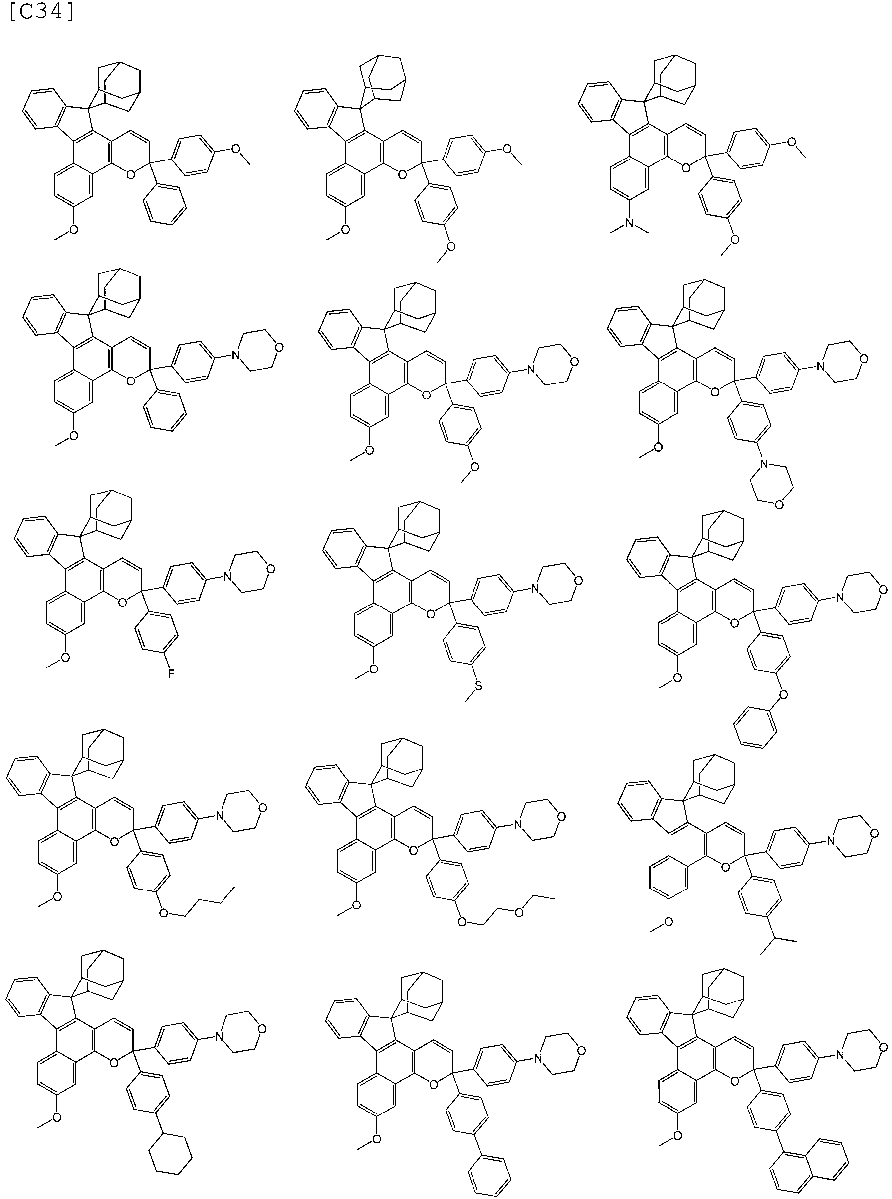

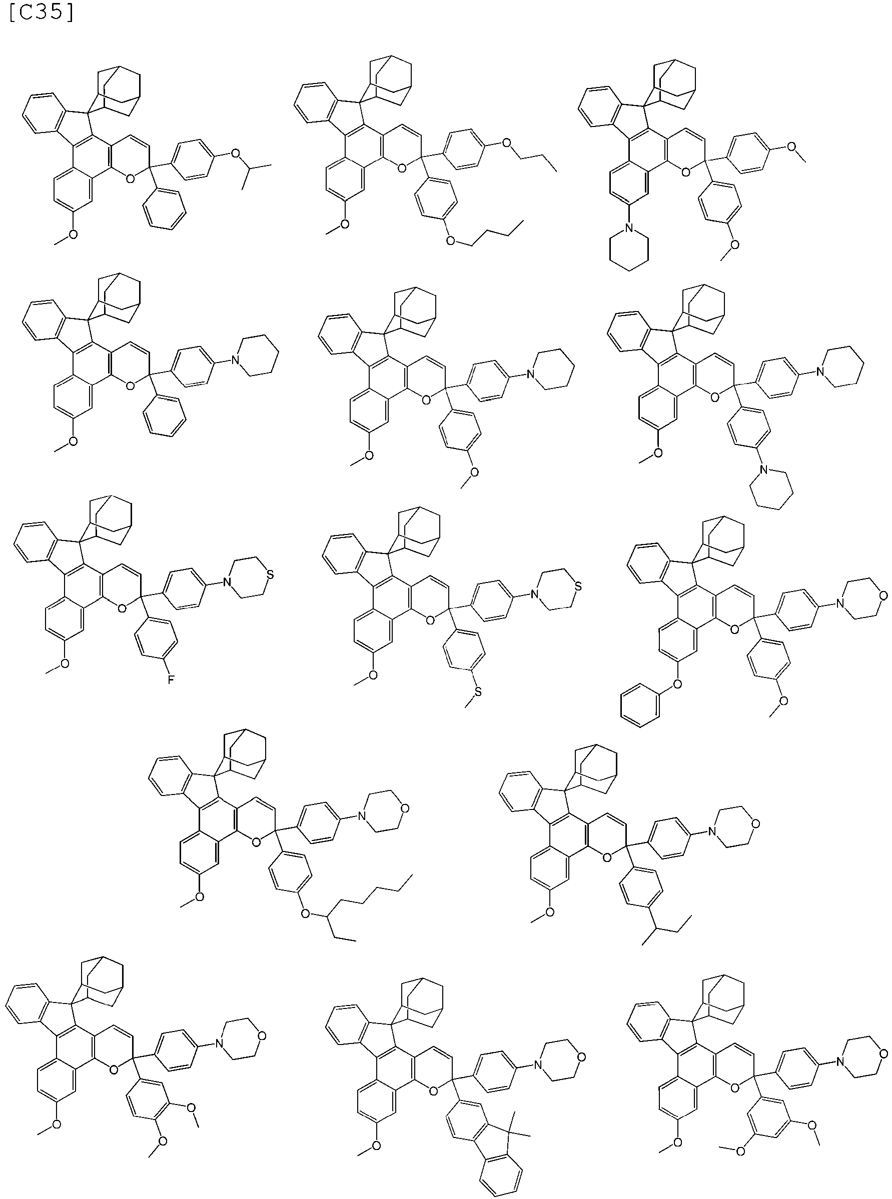

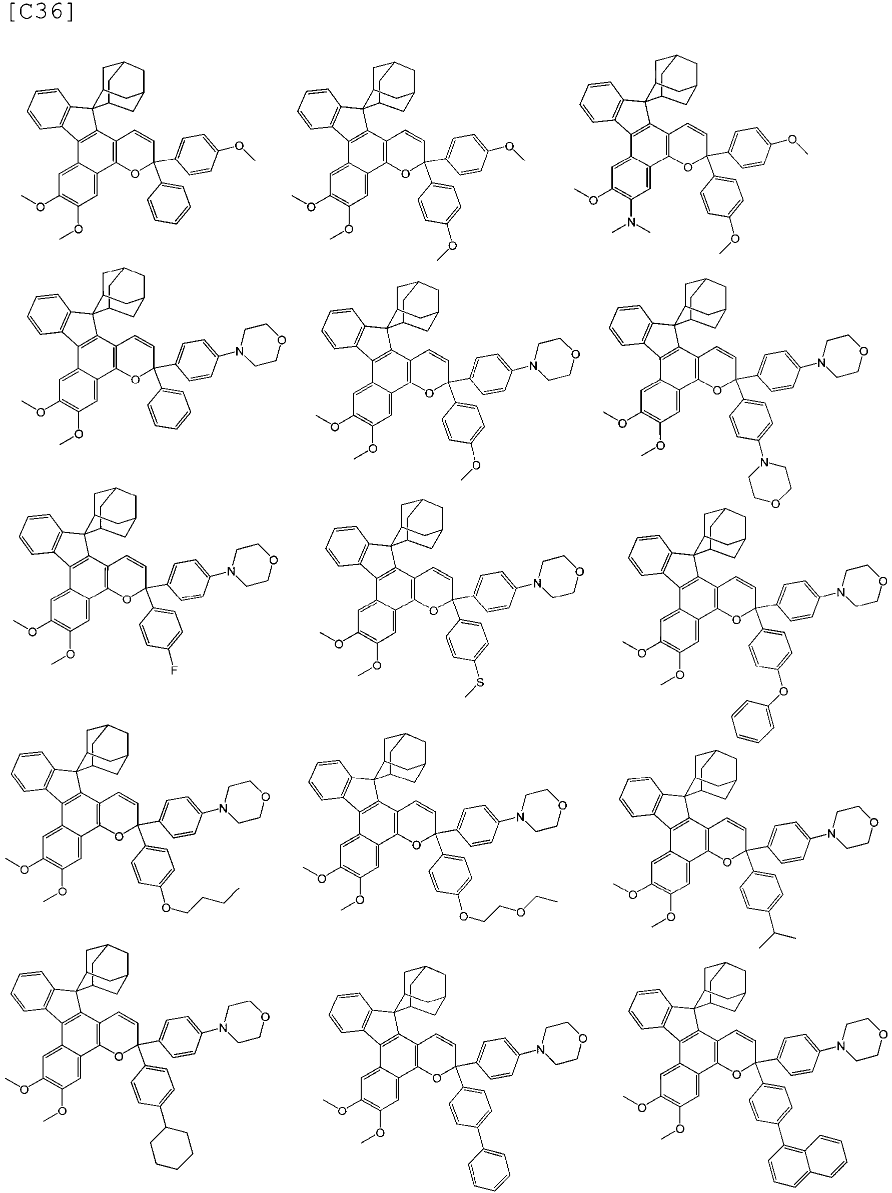

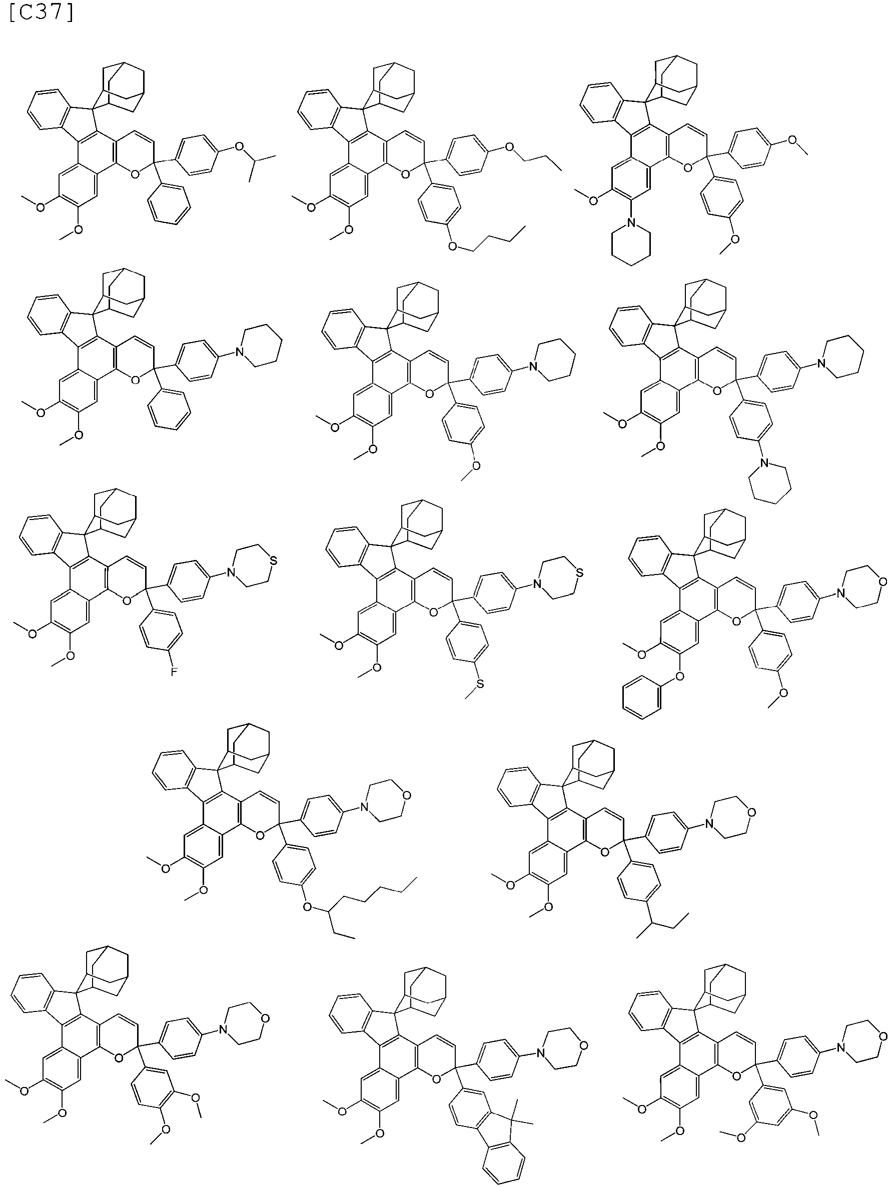

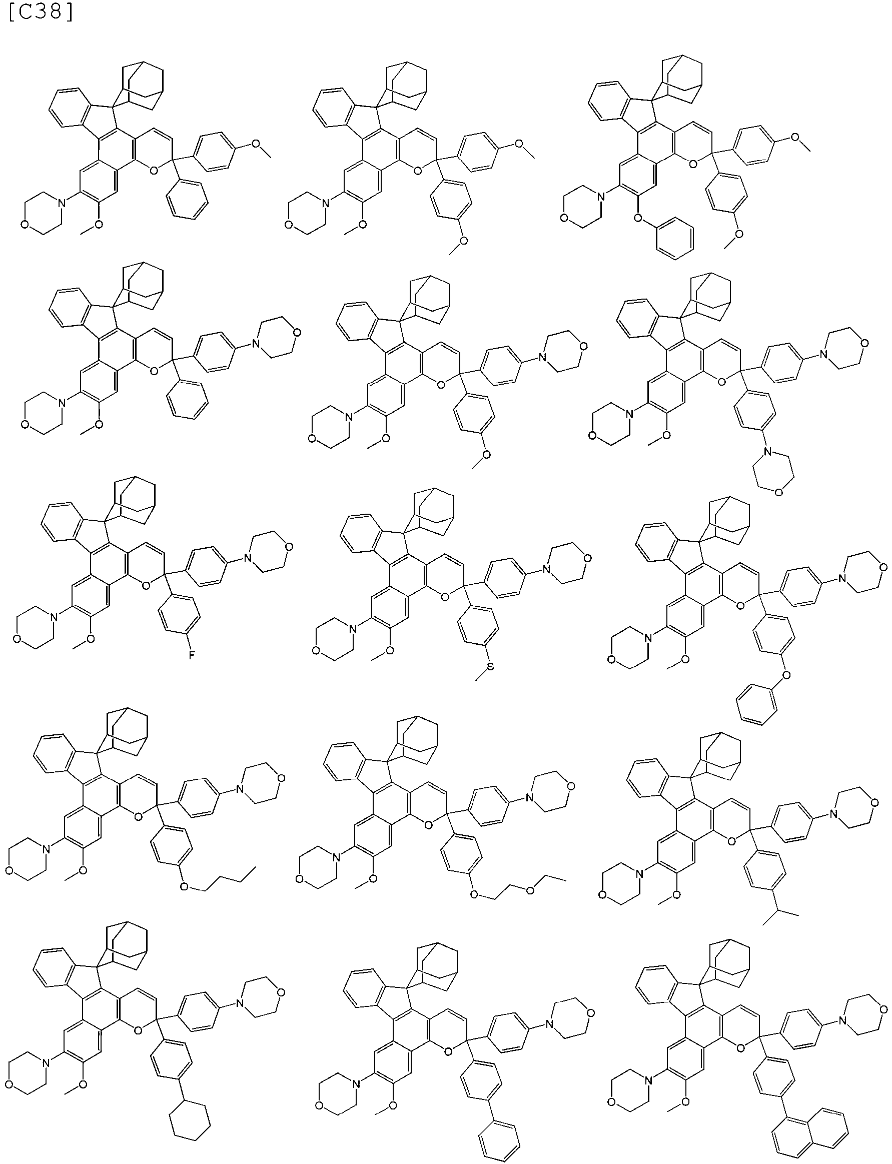

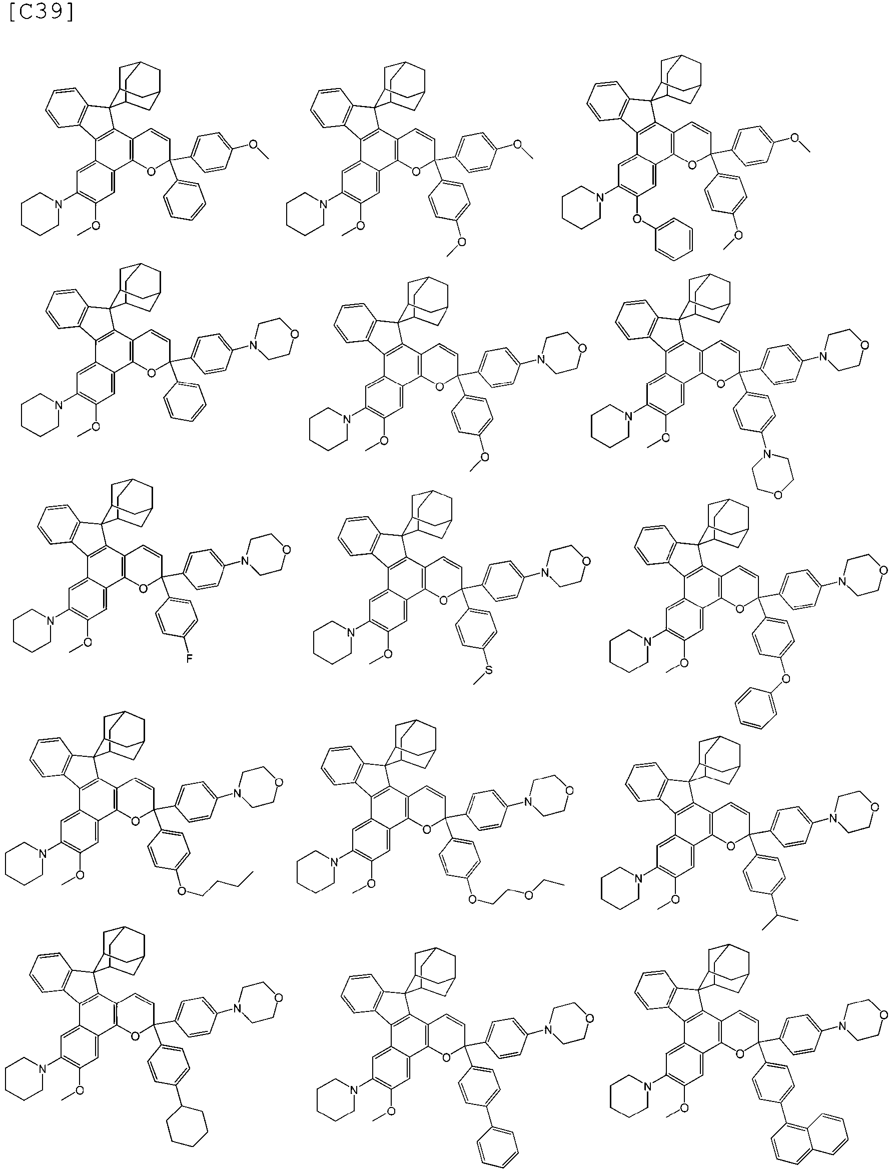

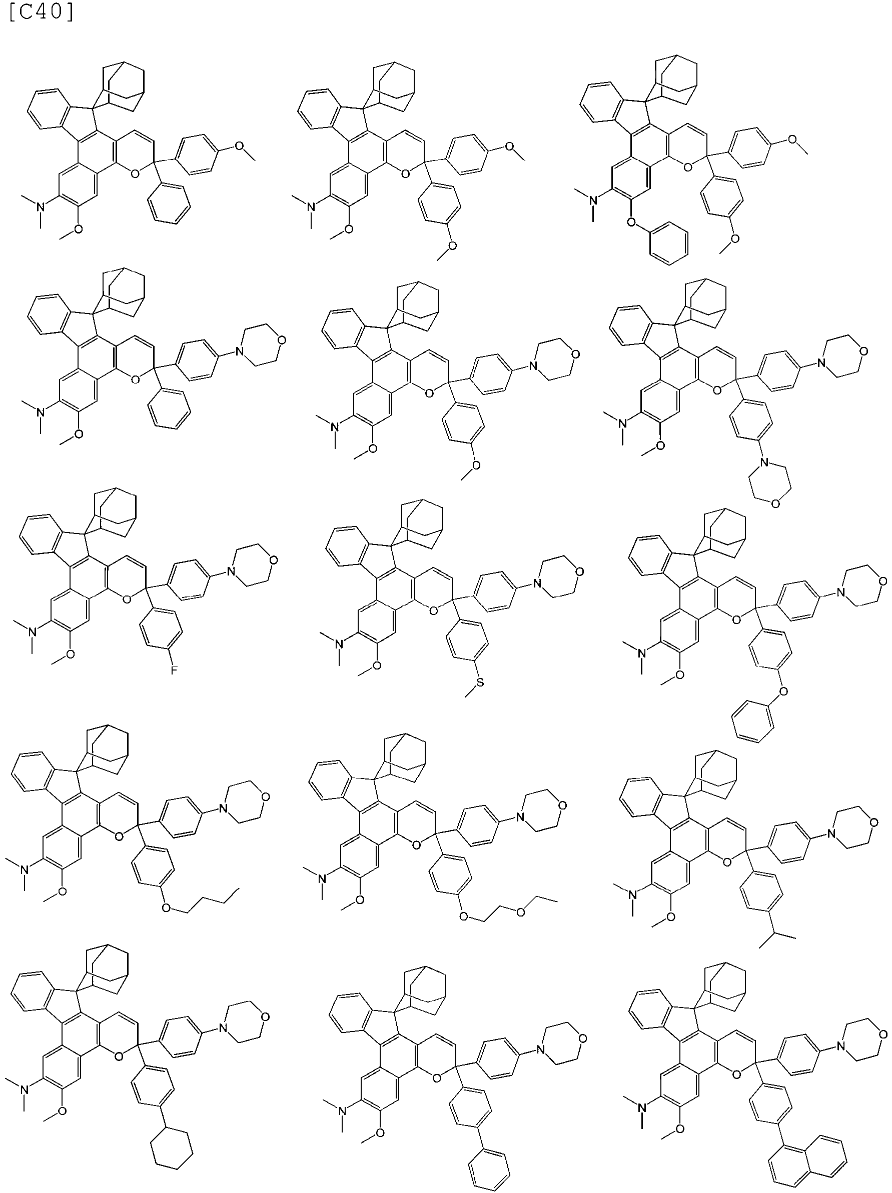

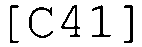

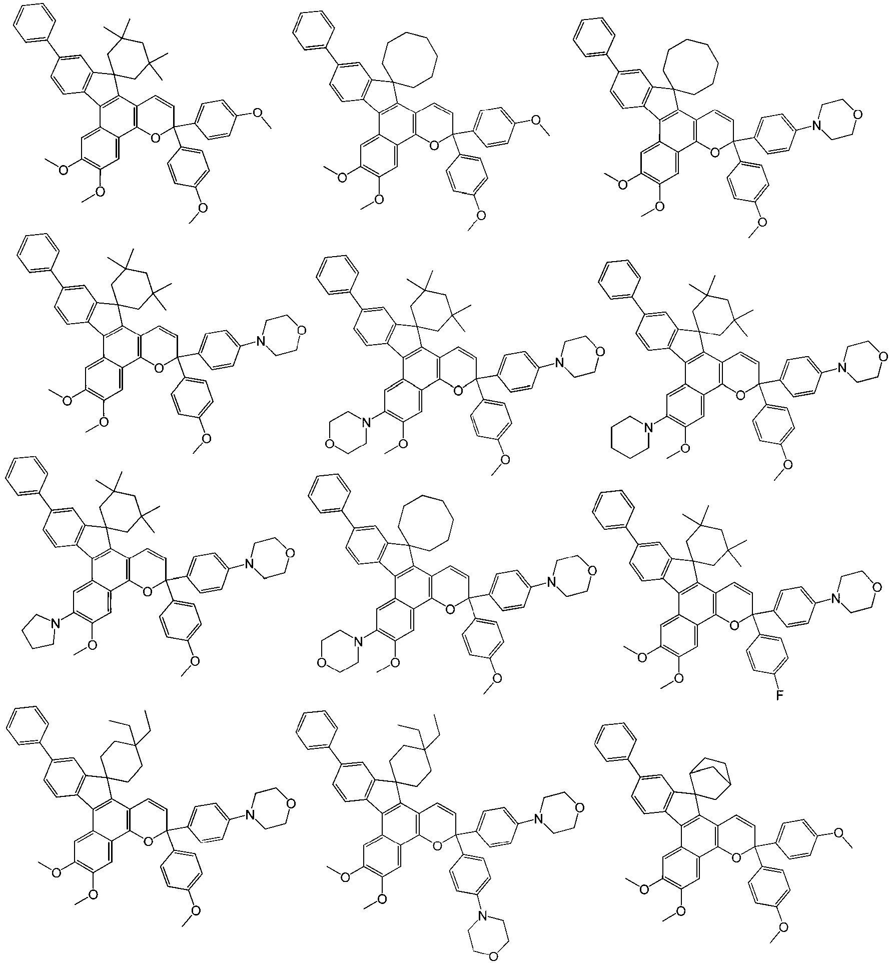

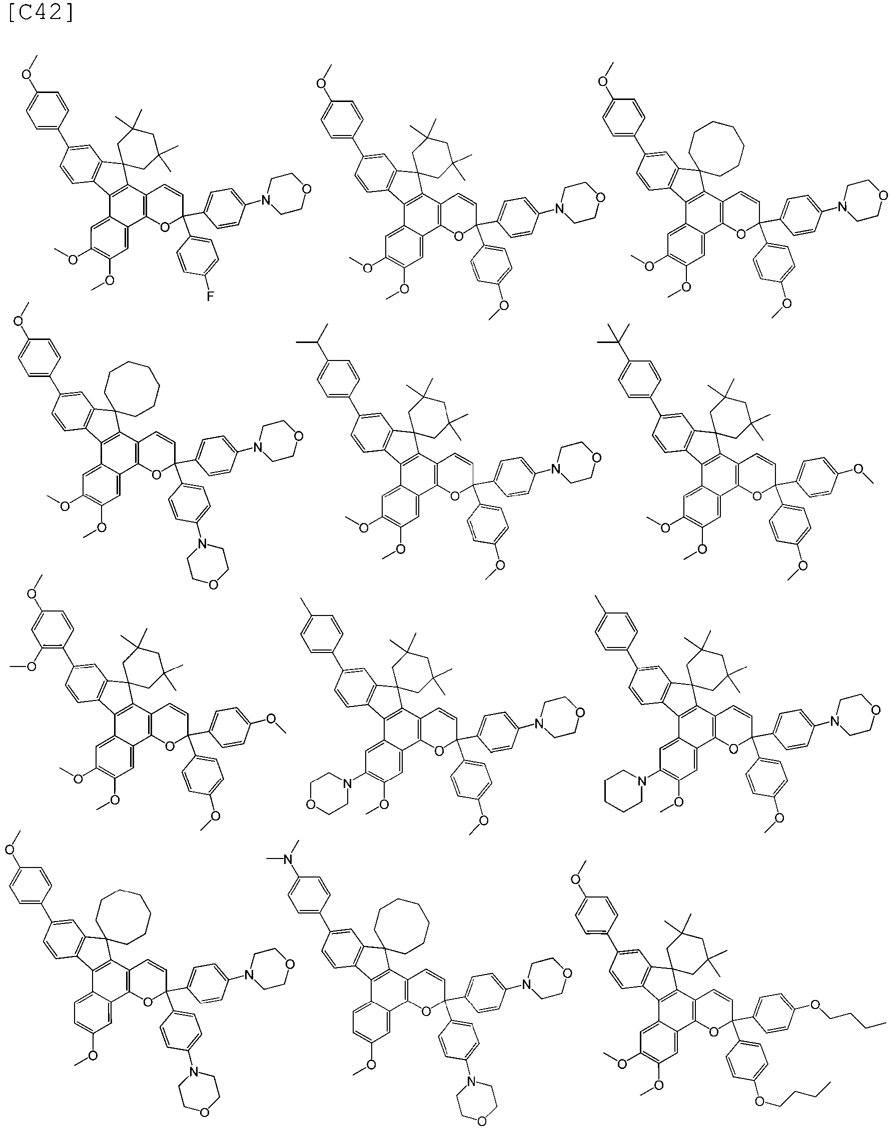

The photochromic compound represented by General Formula 1 can be synthesized by a known method. For the synthesis method, for example, the following documents can be referred to. Japanese Patent No. 4884578, Japanese Patent Application Publication No. 2011-57581, US 2006/0226402 A1, US 2006/0228557 A1, US 2008/0103301 A1, US 2011/0108781 A1, US 2011/0108781 A1, U.S. Patent No. 7527754, U.S. Patent No. 7556751, WO 2001/60811 A1, WO 2013/086248 A1, WO 1996/014596 A1, WO 2001/019813 A1, WO 2001/60811, WO 2009/136668, WO 2010/150905, WO 2011/010744, WO 2011/016582, WO 2011/025056, WO 2011/034202 and WO 2012/102409.

### [Photochromic Composition and Photochromic Article]

One aspect of the present invention relates to a photochromic composition containing one or more photochromic compounds represented by General Formula 1.

In addition, one aspect of the present invention relates to a photochromic article containing one or more photochromic compounds represented by General Formula 1.

The photochromic composition and the photochromic article may contain only one of photochromic compounds represented by General Formula 1 or two or more (for example, two or more and four or less) thereof. The photochromic article and the photochromic composition may contain, for example, about 0.1 to 15.0 mass% of photochromic compounds represented by General Formula 1 with respect to a total amount of 100 mass% thereof. However, the present invention is not limited to the above range.

The photochromic composition and the photochromic article may contain one or more photochromic compounds represented by General Formula 1 and one or more other photochromic compounds. The other photochromic compounds will be described below.

The photochromic article may have at least a substrate. In one aspect, the photochromic compound represented by General Formula 1 may be contained in the substrate of the photochromic article. The photochromic article may have a substrate and a photochromic layer, and the substrate and/or the photochromic layer may contain one or more photochromic compounds represented by General Formula 1. In the substrate and the photochromic layer, in one aspect, the photochromic compound represented by General Formula 1 may be contained only in the substrate, in another aspect, the photochromic compound may be contained only in the photochromic layer, and in still another aspect, the photochromic compound may be contained in the substrate and the photochromic layer. In addition, the substrate and the photochromic layer may contain, as a photochromic compound, only the photochromic compound represented by General Formula 1 or one or more other photochromic compounds. Examples of other photochromic compounds include azobenzenes, spiropyrans, spirooxazines, naphthopyrans, indenonaphthopyrans, phenanthropyrans, hexaarylbisimidazoles, donor-acceptor Stenhouse adducts (DASAs), salicylidene anilines, dihydropyrenes, anthracene dimers, fulgides, diarylethenes, phenoxynaphthacenequinones, and stilbenes. In addition, examples of other photochromic compounds include one or more selected from the group consisting of photochromic compounds represented by General Formula A, photochromic compounds represented by General Formula B and photochromic compounds represented by General Formula C, which are described in WO2022/138966.

### <Substrate>

The photochromic article may contain a substrate selected according to the type of the photochromic article. Examples of substrates include spectacle lens substrates such as a plastic lens substrate and a glass lens substrate. The glass lens substrate may be, for example, a lens substrate made of inorganic glass. Examples of plastic lens substrates include styrene resins such as (meth)acrylic resins, allyl carbonate resins such as a polycarbonate resin, allyl resin, and diethylene glycol bisallyl carbonate resin (CR-39), vinyl resins, polyester resins, polyether resins, urethane resins obtained by reacting an isocyanate compound and a hydroxy compound such as diethylene glycol, thiourethane resins obtained by reacting an isocyanate compound and a polythiol compound, and a cured product obtained by curing a curable composition containing a (thio)epoxy compound having one or more disulfide bonds in the molecule (generally referred to as a transparent resin). As the lens substrate, an undyed substrate (colorless lens) may be used or a dyed substrate (dyed lens) may be used. The index of the lens substrate may be, for example, about 1.50 to 1.75. However, the index of the lens substrate is not limited to the above range, and may be within the above range or may be above or below outside of the above range. Here, the index refers to an index for light having a wavelength of 500 nm. In addition, the lens substrate may be a lens having power (so-called prescription lens) or a lens having no power (so-called non-prescription lens).

For example, the photochromic composition may be a polymerizable composition. In the present invention and this specification, the "polymerizable composition" is a composition containing one or more polymerizable compounds. A polymerizable composition containing at least one or more photochromic compounds represented by General Formula 1 and one or more polymerizable compounds can be molded by a known molding method to produce a cured product of such a polymerizable composition. Such a cured product can be included as a substrate in the photochromic article and/or can be included as a photochromic layer. The curing treatment may be light emission and/or a heat treatment. The polymerizable compound is a compound having a polymerizable group, and as the polymerization reaction of the polymerizable compound proceeds, the polymerizable composition can be cured to form a cured product. The polymerizable composition may further contain one or more additives (for example, a polymerization initiator).

The spectacle lens may include various lenses such as a single focus lens, a multifocal lens, and a progressive power lens. The type of the lens is determined by the surface shape of both sides of the lens substrate. In addition, the surface of the lens substrate may be a convex surface, a concave surface, or a flat surface. In a general lens substrate and spectacle lens, the object-side surface is a convex surface and the eyeball-side surface is a concave surface. However, the present invention is not limited thereto. The photochromic layer may be generally provided on the object-side surface of the lens substrate, or may be provided on the eyeball-side surface.

### <Photochromic Layer>

The photochromic layer may be a layer that is directly provided on the surface of the substrate or indirectly provided via one or more other layers. The photochromic layer may be, for example, a cured layer obtained by curing a polymerizable composition. A photochromic layer can be formed as a cured layer obtained by curing a polymerizable composition containing at least one or more photochromic compounds represented by General Formula 1 and one or more polymerizable compounds. For example, when such a polymerizable composition is directly applied to the surface of the substrate or applied to the surface of the layer provided on the substrate, and a curing treatment is performed on the applied polymerizable composition, a photochromic layer can be formed as a cured layer containing one or more photochromic compounds represented by General Formula 1. As the coating method, known coating methods such as a spin coating method, a dip coating method, a spray coating method, an inkjet method, a nozzle coating method, and a slit coating method can be used. The curing treatment may be light emission and/or a heat treatment. The polymerizable composition may further contain one or more additives (for example, a polymerization initiator) in addition to one or more polymerizable compounds. As the polymerization reaction of the polymerizable compound proceeds, the polymerizable composition can be cured to form a cured layer.

The thickness of the photochromic layer may be, for example, 5 µm or more, 10 µm or more or 20 µm or more, and may be, for example, 80 µm or less, 70 µm or less or 50 µm or less.

### <Polymerizable Compound>

In the present invention and this specification, the term polymerizable compound refers to a compound having one or more polymerizable groups in one molecule, and the term "polymerizable group" refers to a reactive group that can undergo a polymerization reaction. Examples of polymerizable groups include an acryloyl group, methacryloyl group, vinyl group, vinyl ether group, epoxy group, thiol group, oxetane group, hydroxy group, carboxy group, amino group, and isocyanate group.

Examples of polymerizable compounds that can be used to form the above substrate and the above photochromic layer include the following compounds.

### (Episulfide Compound)

The episulfide compound is a compound having two or more episulfide groups in one molecule. The episulfide group is a polymerizable group that can undergo ring-opening polymerization. Specific examples of episulfide compounds include bis(1,2-epithioethyl)sulfide, bis(1,2-epithioethyl)disulfide, bis(2,3-epithiopropyl)sulfide, bis(2,3-epithiopropylthio)methane, bis(2,3-epithiopropyl)disulfide, bis(2,3-epithiopropyldithio)methane, bis(2,3-epithiopropyldithio)ethane, bis(6,7-epithio-3,4-dithiaheptyl)sulfide, bis(6,7-epithio-3,4-dithiaheptyl)disulfide, 1,4-dithiane-2,5-bis(2,3-epithiopropyldithiomethyl), 1,3-bis(2,3-epithiopropyldithiomethyl)benzene, 1,6-bis(2,3-epithiopropyldithiomethyl)-2-(2,3-epithiopropyldithioethylthio)-4-thiahexane, 1,2,3-tris(2,3-epithiopropyldithio)propane, 1,1,1,1-tetrakis(2,3-epithiopropyldithiomethyl)methane, 1,3-bis(2,3-epithiopropyldithio)-2-thiapropane, 1,4-bis(2,3-epithiopropyldithio)-2,3-dithiabutane, 1,1,1-tris(2,3-epithiopropyldithio)methane, 1,1,1-tris(2,3-epithiopropyldithiomethylthio)methane, 1,1,2,2-tetrakis(2,3-epithiopropyldithio)ethane, 1,1,2,2-tetrakis(2,3-epithiopropyldithiomethylthio)ethane, 1,1,3,3-tetrakis(2,3-epithiopropyldithio)propane, 1,1,3,3-tetrakis(2,3-epithiopropyldithiomethylthio)propane, 2-[1,1-bis(2,3-epithiopropyldithio)methyl]-1,3-dithietane, and 2-[1,1-bis(2,3-epithiopropyldithiomethylthio)methyl]-1,3-dithietane.

### (Thietanyl Compound)

The thietanyl compound is a thietane compound having two or more thietanyl groups in one molecule. The thietanyl group is a polymerizable group that can undergo ring-opening polymerization. Some thietanyl compounds have an episulfide group together with a plurality of thietanyl groups. Such compounds are listed as examples in the above episulfide compound. Other thietanyl compounds include metal-containing thietane compounds having metal atoms in the molecule and non-metallic thietane compounds which contain no metal.

Specific examples of non-metallic thietane compounds include bis(3-thietanyl)disulfide, bis(3-thietanyl)sulfide, bis(3-thietanyl)trisulfide, bis(3-thietanyl)tetrasulfide, 1,4-bis(3-thietanyl)-1,3,4-trithiabutane, 1,5-bis(3-thietanyl)-1,2,4,5-tetrathiapentane, 1,6-bis(3-thietanyl)-1,3,4,6-tetrathiahexane, 1,6-bis(3-thietanyl)-1,3,5,6-tetrathiahexane, 1,7-bis(3-thietanyl)-1,2,4,5,7-pentathiaheptane, 1,7-bis(3-thietanylthio)-1,2,4,6,7-pentathiaheptane, 1,1-bis(3-thietanylthio)methane 1,2-bis(3-thietanylthio)ethane, 1,2,3-tris(3-thietanylthio)propane, 1,8-bis(3-thietanylthio)-4-(3-thietanylthiomethyl)-3,6-dithiaoctane, 1,11-bis(3-thietanylthio)-4,8-bis (3-thietanylthiomethyl)-3,6,9-trithiaundecane, 1,11-bis(3-thietanylthio)-4,7-bis(3-thietanylthiomethyl)-3,6,9-trithiaundecane, 1,11-bis(3-thietanylthio)-5,7-bis(3-thietanylthiomethyl)-3,6,9-trithiaundecane, 2,5-bis(3-thietanylthiomethyl)-1,4-dithiane, 2,5-bis[[2-(3-thietanylthio)ethyl]thiomethyl]-1,4-dithiane, 2,5-bis(3-thietanylthiomethyl)-2,5-dimethyl-1,4-dithiane, bisthietanylsulfide, bis(thietanylthio)methane, 3-[<(thietanylthio)methylthio>methylthio]thietane, bisthietanyldisulfide, bisthietanyltrisulfide, bisthietanyltetrasulfide, bisthietanylpentasulfide, 1,4-bis(3-thietanyldithio)-2,3-dithiabutane, 1,1,1-tris(3-thietanyldithio)methane, 1,1,1-tris(3-thietanyldithiomethylthio)methane, 1,1,2,2-tetrakis(3-thietanyldithio)ethane, and 1,1,2,2-tetrakis(3-thietanyldithiomethylthio)ethane.

Examples of metal-containing thietane compounds include those containing Group 14 atoms such as Sn atoms, Si atoms, Ge atoms, and Pb atoms, Group 4 elements such as Zr atoms and Ti atoms, Group 13 atoms such as Al atoms, and Group 12 atoms such as Zn atoms, as metal atoms in the molecule. Specific examples include alkylthio(thietanylthio)tin, bis(alkylthio)bis(thietanylthio)tin, alkylthio(alkylthio)bis(thietanylthio)tin, bis(thietanylthio)cyclic dithiotin compounds, and alkyl(thietanylthio)tin compounds.

Specific examples of alkylthio(thietanylthio)tin include methylthiotris(thietanylthio)tin, ethylthiotris(thietanylthio)tin, propylthiotris(thietanylthio)tin, and isopropylthiotris(thietanylthio)tin.

Specific examples of bis(alkylthio)bis(thietanylthio)tin include bis(methylthio)bis(thietanylthio)tin, bis(ethylthio)bis(thietanylthio)tin, bis(propylthio)bis(thietanylthio)tin, and bis(isopropylthio)bis(thietanylthio)tin.

Specific examples of alkylthio(alkylthio)bis(thietanylthio)tin include ethylthio(methylthio)bis(thietanylthio)tin, methylthio(propylthio)bis(thietanylthio)tin, isopropylthio(methylthio)bis(thietanylthio)tin, ethylthio(propylthio)bis(thietanylthio)tin, ethylthio(isopropylthio)bis(thietanylthio)tin, and isopropylthio(propylthio)bis(thietanylthio)tin.

Specific examples of bis(thietanylthio)cyclic dithiotin compounds include bis(thietanylthio)dithiastannetane, bis(thietanylthio)dithiastannolane, bis(thietanylthio)dithiastanninane, and bis(thietanylthio)trithiastannocane.

Specific examples of alkyl(thietanylthio)tin compounds include methyltris(thietanylthio)tin, dimethyl bis(thietanylthio)tin, butyltris(thietanylthio)tin, and tetrakis(thietanylthio)tin.

### (Polyamine Compound)

The polyamine compound is a compound having two or more NH₂ groups in one molecule, and can form a urea bond according to a reaction with a polyisocyanate and can form a thiourea bond according to a reaction with a polyisothiocyanate. Specific examples of polyamine compounds include ethylenediamine, hexamethylenediamine, isophoronediamine, nonamethylenediamine, undecamethylenediamine, dodecamethylenediamine, metaxylenediamine, 1,3-propanediamine, putrescine, 2-(2-aminoethylamino)ethanol, diethylenetriamine, p-phenylenediamine, m-phenylenediamine, melamine, and 1,3,5-benzenetriamine.

### (Epoxy Compound)

The epoxy compound is a compound having an epoxy group in the molecule. The epoxy group is a polymerizable group that can undergo ring-opening polymerization. The epoxy compounds are generally classified into aliphatic epoxy compounds, alicyclic epoxy compounds and aromatic epoxy compounds.

Specific examples of aliphatic epoxy compounds include ethylene oxide, 2-ethyloxirane, butyl glycidyl ether, phenyl glycidyl ether, 2,2'-methylenebisoxirane, 1,6-hexanediol diglycidyl ether, ethylene glycol diglycidyl ether, diethylene glycol diglycidyl ether, triethylene glycol diglycidyl ether, tetraethylene glycol diglycidyl ether, nonaethylene glycol diglycidyl ether, propylene glycol diglycidyl ether, dipropylene glycol diglycidyl ether, tripropylene glycol diglycidyl ether, tetrapropylene glycol diglycidyl ether, nonapropylene glycol diglycidyl ether, neopentyl glycol diglycidyl ether, trimethylolpropane triglycidyl ether, glycerol triglycidyl ether, diglycerol tetraglycidyl ether, pentaerythritol tetraglycidyl ether, and tris(2-hydroxyethyl)isocyanurate triglycidyl ether.

Specific examples of alicyclic epoxy compounds include isophoronediol diglycidyl ether and bis-2,2-hydroxycyclohexylpropane diglycidyl ether.

Specific examples of aromatic epoxy compounds include resorcine diglycidyl ether, bisphenol A diglycidyl ether, bisphenol F diglycidyl ether, bisphenol S diglycidyl ether, orthophthalic acid diglycidyl ester, phenol novolac polyglycidyl ether, and cresol novolac polyglycidyl ether.

In addition, in addition to the above examples, an epoxy compound having a sulfur atom in the molecule together with an epoxy group can be used. Such epoxy compounds containing sulfur atoms include linear aliphatic compounds and cycloaliphatic compounds.

Specific examples of linear aliphatic sulfur atom-containing epoxy compounds include bis(2,3-epoxypropyl)sulfide, bis(2,3-epoxypropyl)disulfide, bis(2,3-epoxypropylthio)methane, 1,2-bis(2,3-epoxypropylthio)ethane, 1,2-bis(2,3-epoxypropylthio)propane, 1,3-bis(2,3-epoxypropylthio)propane, 1,3-bis(2,3-epoxypropylthio)-2-methylpropane, 1,4-bis(2,3-epoxypropylthio)butane, 1,4-bis(2,3-epoxypropylthio)-2-methylbutane, 1,3-bis(2,3-epoxypropylthio)butane, 1,5-bis(2,3-epoxypropylthio)pentane, 1,5-bis(2,3-epoxypropylthio)-2-methylpentane, 1,5-bis(2,3-epoxypropylthio)-3-thiapentane, 1,6-bis(2,3-epoxypropylthio)hexane, 1,6-bis(2,3-epoxypropylthio)-2-methylhexane, 3,8-bis(2,3-epoxypropylthio)-3,6-dithiaoctane, 1,2,3-tris(2,3-epoxypropylthio)propane, 2,2-bis(2,3-epoxypropylthio)-1,3-bis(2,3-epoxypropylthiomethyl)propane, and 2,2-bis(2,3-epoxypropylthiomethyl)-1-(2,3-epoxypropylthio)butane.

Specific examples of cyclic aliphatic sulfur atom-containing epoxy compounds include 1,3-bis(2,3-epoxypropylthio)cyclohexane, 1,4-bis(2,3-epoxypropylthio)cyclohexane, 1,3-bis(2,3-epoxypropylthiomethyl)cyclohexane, 1,4-bis(2,3-epoxypropylthiomethyl)cyclohexane, 2,5-bis(2,3-epoxypropylthiomethyl)-1,4-dithiane, 2,5-bis[<2-(2,3-epoxypropylthio)ethyl>thiomethyl]-1,4-dithiane, and 2,5-bis(2,3-epoxypropylthiomethyl)-2,5-dimethyl-1,4-dithiane.

### (Compound Having Radically Polymerizable Group)

The radically polymerizable group is a polymerizable group that can undergo radical polymerization. Examples of radically polymerizable groups include an acryloyl group, methacryloyl group, allyl group, and vinyl group.

In the following, a compound having a polymerizable group selected from the group consisting of acryloyl groups and methacryloyl groups will be referred to as a "(meth)acrylate compound". Specific examples of (meth)acrylate compounds include ethylene glycol di(meth)acrylate, diethylene glycol di(meth)acrylate, triethylene glycol di(meth)acrylate, tetraethylene glycol di(meth)acrylate, polyethylene glycol di(meth)acrylate, propylene glycol di(meth)acrylate, dipropylene glycol di(meth)acrylate, tripropylene glycol di(meth)acrylate, polypropylene glycol di(meth)acrylate, neopentyl glycol di(meth)acrylate, ethylene glycol bisglycidyl(meth)acrylate, bisphenol A di(meth)acrylate, 2,2-bis(4-(meth)acryloyloxyethoxyphenyl)propane, 2,2-bis(4-(meth) acryloxydiethoxyphenyl) propane, 2,2-bis(4-(meth)acryloyloxyethoxyphenyl)propane, 2,2-bis(3,5-dibromo-4-(meth)acryloyloxyethoxyphenyl)propane, 2,2-bis(4-(meth)acryloyloxydipropoxyphenyl)propane, bisphenol F di(meth)acrylate, 1,1-bis(4-(meth)acryloyloxyethoxyphenyl)methane, 1,1-bis(4-(meth)acryloxydiethoxyphenyl)methane, dimethylol tricyclodecane di(meth)acrylate, trimethylolpropane tri(meth)acrylate, ditrimethylolpropanetetra(meth)acrylate, glycerol di(meth)acrylate, pentaerythritol tri(meth)acrylate, pentaerythritol tetra(meth)acrylate, methylthio(meth)acrylate, phenylthio(meth)acrylate, benzylthio(meth)acrylate, xylylenedithiol di(meth)acrylate, mercaptoethyl sulfide di(meth)acrylate, and bifunctional urethane (meth)acrylate.

Specific examples of compounds having an allyl group (allyl compounds) include allyl glycidyl ether, diallyl phthalate, diallyl terephthalate, diallyl isophthalate, diallyl carbonate, diethylene glycol bisallyl carbonate, methoxypolyethylene glycol allyl ether, polyethylene glycol allyl ether, methoxypolyethyleneglycol-polypropylene glycol allyl ether, butoxy polyethylene glycol-polypropylene glycol allyl ether, methacryloyloxy polyethylene glycol-polypropylene glycol allyl ether, phenoxy polyethylene glycol allyl ether, and methacryloyloxy polyethylene glycol allyl ether.

Examples of compounds having a vinyl group (vinyl compounds) include α-methylstyrene, α-methylstyrene dimer, styrene, chlorostyrene, methylstyrene, bromostyrene, dibromostyrene, divinylbenzene, and 3,9-divinylspirobi(m-dioxane).

The photochromic article may include one or more layers known as functional layers of the photochromic article such as a protective layer for improving the durability of the photochromic article, an anti-reflection layer, a water-repellent or hydrophilic anti-fouling layer, an anti-fogging layer, and a primer layer for improving adhesion between layers at any position.

The photochromic article may be an optical article. One form of the optical article is a spectacle lens. Such a spectacle lens can also be called a photochromic lens or a photochromic spectacle lens. In addition, as one form of the optical article, a goggle lens, a visor (cap) part of a sun visor, a shield component of a helmet and the like may be exemplified. The photochromic composition which is a polymerizable composition is applied to the substrate for these optical articles, a curing treatment is performed on the applied composition, a photochromic layer is formed, and thereby an optical article having an anti-glare function can be obtained.

### [Spectacles]

One aspect of the present invention relates to spectacles having a spectacle lens that is one form of the photochromic article. Details of the spectacle lens included in the spectacles are as described above. By providing such a spectacle lens, for example, the spectacles can exhibit an anti-glare effect like sunglasses when the photochromic compound is colored upon receiving sunlight outdoors, and the photochromic compound can fade upon returning indoors, and thus the transmittance can be recovered. For the spectacles, a known technique can be applied to the configuration of the frame or the like.

### [Examples]

Hereinafter, the present invention will be described in more detail with reference to examples. However, the present invention is not limited to embodiments shown in examples.

In the following, the molecular structure was identified using a nuclear magnetic resonance device (NMR). Proton NMR of ECS-400 (commercially available from JEOL Ltd.) was used as NMR. As a measurement solvent, deuterated chloroform was mainly used, and heavy dimethylsulfoxide, heavy acetone, heavy acetonitrile, heavy benzene, heavy methanol, heavy pyridine or the like was appropriately used only when it was poorly soluble in deuterated chloroform.

The purity was analyzed using high-performance liquid chromatography (HPLC). LC-2040C (commercially available from Shimadzu Corporation) was used as HPLC. YMC-Triart C18 was used for the column, and the measurement temperature was set to 40°C. For the mobile phase, a mixed solvent containing water containing 0.1% of trifluoroacetic acid and acetonitrile was used and the flow rate was 0.4 mL/min.

For mass spectrometry, a device including SQD2 was used as a mass spectrometry unit in ACQUITY UPLC H-Class system (UPLC) (commercially available from Nihon Waters K.K.). ACQUITY UPLC BEH C18 was used as the column, and the measurement temperature was set to 40°C. For the mobile phase, a mixed solvent containing water to which formic acid was added and acetonitrile was used, a concentration gradient was applied and a flow rate was set to 0.61 mL/min for flowing. An electrospray ionization (ESI) method was used for ionization.

CHN (carbon·hydrogen·nitrogen) elemental analysis was performed by a combustion method.

### [Example 1]

From the reaction product shown in the following table, a product shown in the following table was obtained by the following method.

Under an argon atmosphere, p-toluenesulfonic acid monohydrate (0.15 g, 0.80 mmol) was added to a toluene solution (36 mL) containing Reaction Product 1 (1.5 g, 4 mmol) and Reaction Product 2 (2.3 g, 8 mmol) shown in the following table, and the mixture was stirred at room temperature overnight. An aqueous sodium hydroxide solution (1.0 M, 37 mL) was added thereto, and the mixture was stirred for about 20 minutes. Impurities removed by filtration, extraction with toluene (30 mL×2) was performed, the combined organic layer was then washed with water (20 mL×2) and concentrated. The obtained residue was purified through column chromatography (SiO₂: 200 g, heptane/chloroform (volume basis)=70/30 to 60/40) to obtain 1.0 g of a brown solid. The obtained solid was suspended in heptane/ethyl acetate (2/1 (volume basis), 90 mL), subjected to an ultrasonic treatment for about 30 minutes, filtered and dried, and as the final product, a product shown in the following table was obtained as a light blue solid (0.8 g).

The obtained products were analyzed by the following method.

The structure was identified by a nuclear magnetic resonance device (NMR).

The purity was analyzed by HPLC and was a value shown in the following table in terms of area ratio.

As a result of mass spectrometry, the measured value ([M+H]⁺, relative intensity 100) was shown in the following table for the calculated value of the exact mass shown in the following table.

As a result of CHN elemental analysis by a combustion method, the measured value was the value shown in the following table for the calculated value shown in the following table.

It was confirmed on the basis of the above analysis results that compounds shown in the following table as desired compounds were obtained comprehensively.

### [Examples 2 to 6 and Comparative Examples 1 and 2]

Compounds shown in the following table were obtained by the same operation as above, except that reaction products shown in the following table were used as Reaction Product 1 and Reaction Product 2 used to synthesize the compound represented by General Formula 1.

The obtained products were analyzed by the method described above. The analysis results are shown in the following table.

### [Production of Spectacle Lens (Photochromic Article)]

### <Preparation of Photochromic Composition (Polymerizable Composition)>

In a plastic container, with respect to a total amount of 100 parts by mass of (meth)acrylates, 68 parts by mass of polyethylene glycol diacrylate, 12 parts by mass of trimethylolpropane trimethacrylate, and 20 parts by mass of neopentyl glycol dimethacrylate were mixed to prepare a (meth)acrylate mixture. With respect to 100 parts by mass of the (meth)acrylate mixture, 2.5 parts by mass of each photochromic compound of Examples 1 to 6 was mixed. In addition, a photopolymerization initiator (phenyl bis(2,4,6-trimethylbenzoyl)phosphine oxide), an antioxidant [bis(3-tert-butyl-4-hydroxy-5-methylphenyl)propionate)][ethylene bis(oxyethylene) and a light stabilizer (bis(1,2,2,6,6-pentamethyl-4-piperidyl)sebacate) were mixed and sufficiently stirred and a silane coupling agent (γ-methacryloxypropyltrimethoxysilane) was then added dropwise with stirring. Then, defoaming was performed using an automatic revolution type stirring and defoaming device.

A photochromic composition was prepared by the above method.

### <Formation of Primer Layer>

A plastic lens substrate (commercially available from HOYA, product name EYAS: a center thickness of 2.5 mm, a diameter of 75 mm, and a spherical lens power of -4.00) was immersed in an aqueous sodium hydroxide solution having a concentration of 10 mass% (a liquid temperature of 60°C) for 5 minutes, washed with an alkali and additionally washed with pure water and dried. Then, a water-based polyurethane resin liquid (polycarbonate polyol-based polyurethane emulsion, a viscosity of 100 cPs, and a solid content concentration of 38 mass%) was applied to the convex surface of the plastic lens substrate in an environment of room temperature and a relative humidity of 40 to 60% using a spin coater MS-B150 (commercially available from Mikasa Corporation) at a rotational speed of 1,500 rpm for 1 minute according to a spin coating method and then dried naturally for 15 minutes, and thereby a primer layer having a thickness of 5.5 µm was formed.

### <Formation of Photochromic Layer>

The photochromic composition prepared above was added dropwise to the primer layer, and applied by a spin coating method using a program in which the rotational speed was changed in a slope mode from a rotational speed of 500 rpm to 1,500 rpm over 1 minute, and rotation was additionally performed at 1,500 rpm for 5 seconds using MS-B150 (commercially available from Mikasa Corporation). Then, ultraviolet light (with a dominant wavelength of 405 nm) was emitted to the photochromic composition applied on the primer layer formed on the plastic lens substrate in a nitrogen atmosphere (with an oxygen concentration of 500 ppm or less) for 40 seconds, and the composition was cured to form a photochromic layer. The thickness of the formed photochromic layer was 45 µm.

Accordingly, photochromic articles (spectacle lenses) containing the photochromic compounds of Examples 1 to 6 were produced. It was confirmed that all of the spectacle lenses were colored when exposed to ultraviolet light and returned to their original state before exposure to ultraviolet light when the exposure to ultraviolet light was stopped.

### [Evaluation Method]

### <Evaluation of Fading Rate>

For examples and comparative examples shown in the following table, each compound was dissolved in chloroform containing no stabilizer to prepare a chloroform solution containing the compound.

A 1 cm square quartz spectroscopic cell containing the prepared solution was covered, and ultraviolet light was emitted using a UV-LED (a combination of LIGHTNINGCURE LC-L1V5 and L14310-120, an output of 70%, commercially available from Hamamatsu Photonics K.K.) as an ultraviolet light source for 15 seconds. The solution was stirred with a small stirrer during ultraviolet light emission. Within 10 seconds after ultraviolet light emission was completed, the absorbance was measured using a UV-visible spectrophotometer (UV-1900i, commercially available from Shimadzu Corporation, a measurement wavelength of 700 to 400 nm, wavelength increments of 2 nm, survey mode). The absorbance was measured at room temperature (23 to 28°C). Here, the concentration of the solution was prepared so that the absorbance at the first absorption wavelength (the peak of the absorption intensity observed at the longest wavelength) was 0.95 to 1.05. In addition, the absorbance was measured every 10 seconds, and the attenuation of the absorbance was measured. Normalization was performed so that the peak of the first absorption wavelength in the first absorbance measurement became 1, the attenuation of the absorbance was then measured, data of fading for an initial 100 seconds (11 absorbance measurements) from the change in absorbance over time was analyzed with a first-order reaction model, and thus the reaction rate constant was obtained. If [A₀] is the initial concentration of the colored component, that is, the normalized absorbance value of 1, [A] is the concentration of the colored component after a certain time, that is, the normalized absorbance value, t is the time (seconds), and k is a rate constant, the first-order reaction can be expressed as in the following formula.

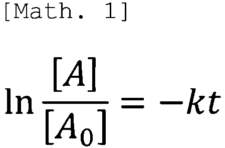

### <Evaluation of Transparency in Visible Range>

Each compound of examples and comparative examples was dissolved in chloroform containing no stabilizer to prepare a chloroform solution containing the compound (concentration: 2.8×10⁻³ mol/L).

A 1 cm square quartz spectroscopic cell containing the prepared solution was covered, and left in a UV-visible spectrophotometer (that is, under light-shielded conditions) for 30 minutes, and the absorbance was then measured using a UV-visible spectrophotometer (UV-1900i, commercially available from Shimadzu Corporation, measurement wavelength: 800 to 250 nm, wavelength increments of 2 nm, high-speed mode). The absorbance was measured at room temperature (20 to 25°C).

The molar absorption coefficient ε (mol⁻¹Lcm⁻¹) at a wavelength of 400 nm was read. When the molar absorption coefficient ε was less than 8,000 mol⁻¹Lcm⁻¹, it was determined as "satisfactory", and when the molar absorption coefficient ε was 8,000 mol⁻¹Lcm⁻¹ or more, it was determined as "unsatisfactory".

The following table shows the evaluation results of the reaction rate constant and the visible range transparency obtained for the examples and comparative examples. On the basis of the results shown in the following table, it was confirmed that the compounds of the examples shown in the following table exhibited a faster fading rate than the compound of Comparative Example 2 and better visible range transparency than the compound of Comparative Example 1.

In addition, in the above measurement, it was confirmed that all of the compounds of the examples exhibited photochromic performance in which the structure was transformed into a molecular structure with strong visible range absorption when ultraviolet light was emitted.

**[Table 1-1]**

| | Reaction Product 1 | Reaction Product 2 | Compound (product) |
|---|---|---|---|
| Example1 | 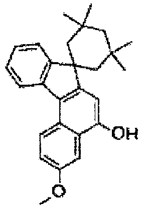 | 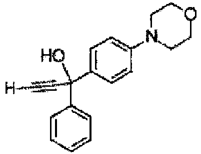 | 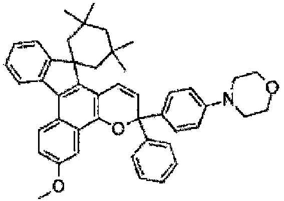 |
| Example2 | 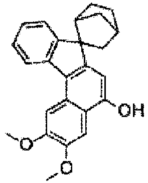 | 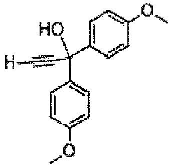 | 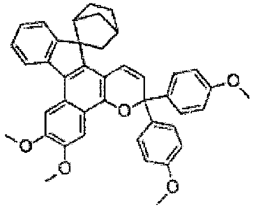 |
| Example3 | 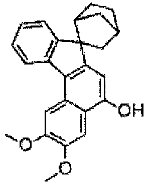 | 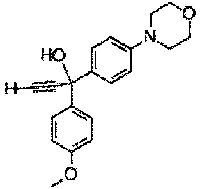 | 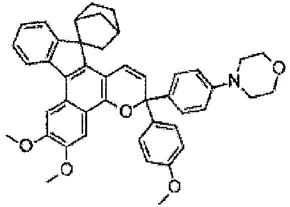 |

**[Table 1-2]**

| | Reaction Product 1 | Reaction Product 2 | Compound (product) |
|---|---|---|---|
| Example4 | 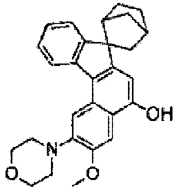 | 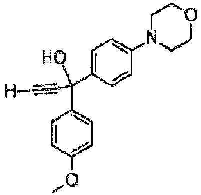 | 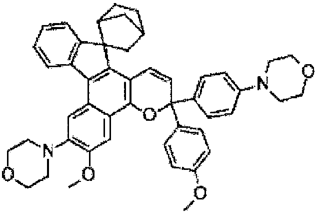 |
| Example5 | 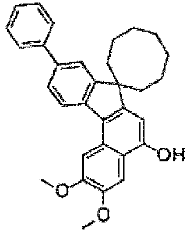 | 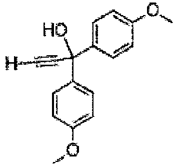 | 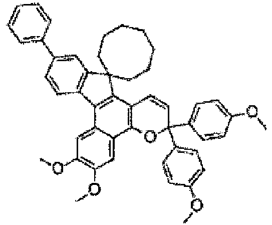 |
| Example6 | 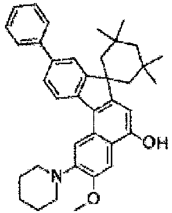 | 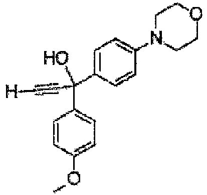 | 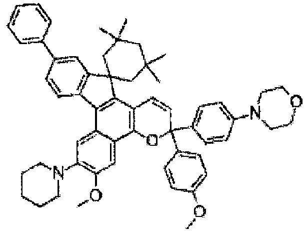 |

**[Table 1-3]**

| | Reaction Product 1 | Reaction Product 2 | Compound (product) |
|---|---|---|---|
| Comparative Example1 | 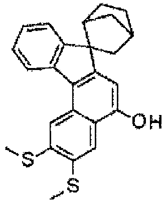 | 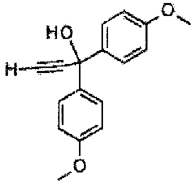 | 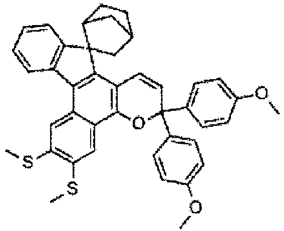 |
| Comparative Example2 | 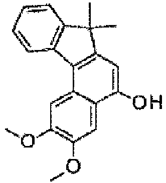 | 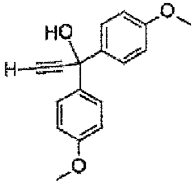 | 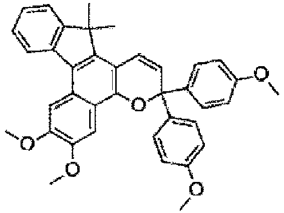 |

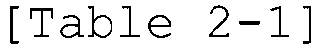

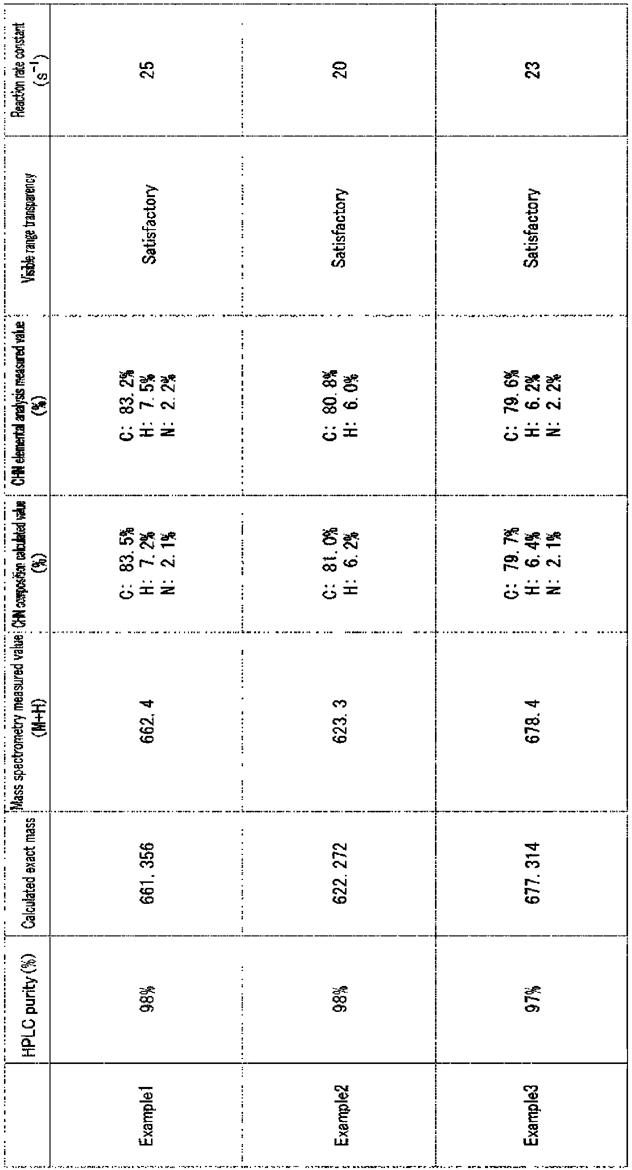

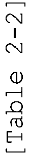

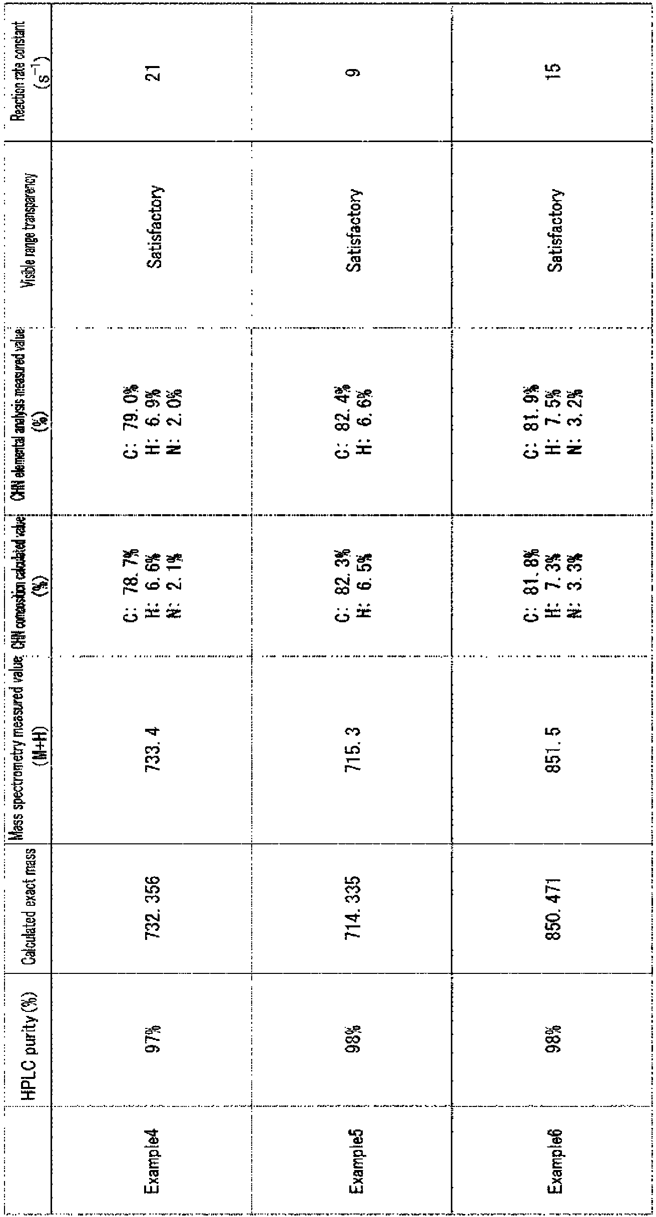

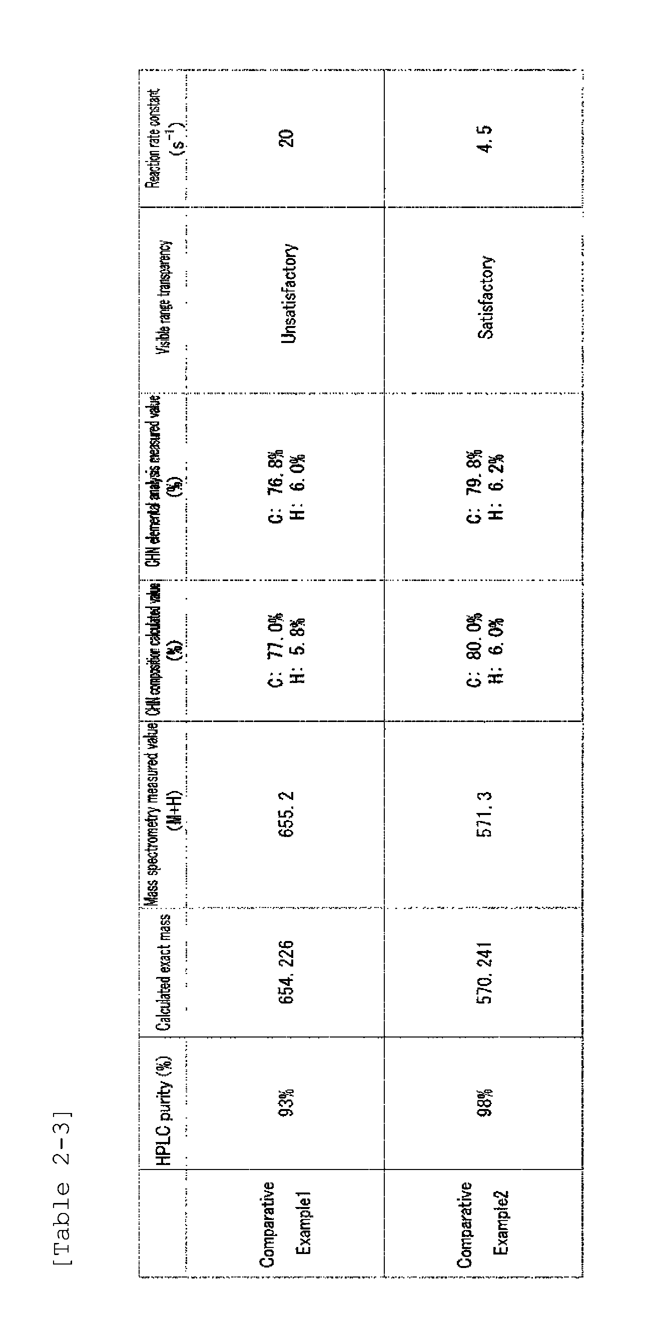

Finally, the above aspects are summarized.

[1] A photochromic compound represented by the following General Formula 1.
   [C43] 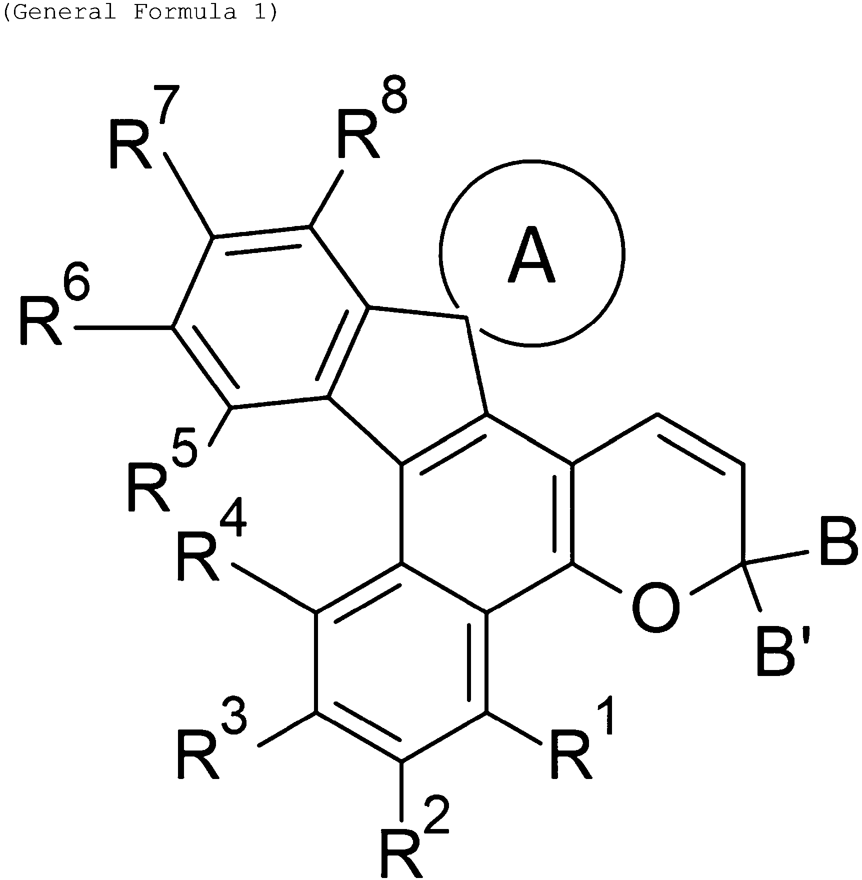 (in General Formula 1,
   A represents a ring structure having 3 or more carbon atoms constituting a ring including the carbon atom at position 13 of indeno-fused naphthopyran,
   R¹ and R⁴ to R⁸ each independently represent a hydrogen atom or a substituent,
   R² represents an electron-donating group (excluding a sulfur-containing electron-donating group),
   R³ represents a hydrogen atom or an electron-donating group (excluding a sulfur-containing electron-donating group), and
   B and B' each independently represent a substituent).
[2] The photochromic compound according to [1], wherein R² represents an alkoxy group and R³ represents a hydrogen atom.
[3] The photochromic compound according to [1], wherein R² represents an alkoxy group or a phenoxy group and R³ represents an amino group.
[4] The photochromic compound according to [3], wherein R² represents an alkoxy group and R³ represents an amino group.
[5] The photochromic compound according to [4], wherein the alkoxy group represented by R² is a methoxy group.
[6] The photochromic compound according to any one of [3] to [5], wherein the amino group represented by R³ is a morpholino group or a piperidino group.
[7] The photochromic compound according to any one of [1] to [6], wherein R⁷ represents an aryl group.
[8] The photochromic compound according to [7], wherein the aryl group represented by R⁷ is a phenyl group.
[9] The photochromic compound according to [1], wherein R² and R³ each independently represent an alkoxy group.
[10] The photochromic compound according to [9], wherein R² and R³ each represent a methoxy group.
[11] The photochromic compound according to [9] or [10], wherein R⁷ represents an aryl group.
[12] The photochromic compound according to [11], wherein the aryl group represented by R⁷ is a phenyl group.
[13] The photochromic compound according to any one of [1] to [12], wherein the ring structure represented by A is an aliphatic ring.
[14] The photochromic compound according to [13], wherein the number of carbon atoms constituting the aliphatic ring is 3 or more and 6 or less.
[15] The photochromic compound according to [13], wherein the number of carbon atoms constituting the aliphatic ring is 7 or more and 20 or less.
[16] The photochromic compound according to any one of [1] to [15], wherein at least one of B and B' represents a phenyl group having a substituent at a substitution position para to the carbon atom at the position bonding to a pyran ring of indeno-fused naphthopyran.
[17] The photochromic compound according to any one of [1] to [16], wherein B and B' each independently represent a phenyl group having an electron-donating group as a substituent at a substitution position para to the carbon atom at the position bonding to a pyran ring of indeno-fused naphthopyran.
[18] The photochromic compound according to any one of [1] to [17], wherein R¹, R⁴, R⁵, R⁶ and R⁸ each represent a hydrogen atom.
[19] A photochromic composition including the photochromic compound according to any one of [1] to [18].
[20] The photochromic composition according to [19], including one or more of the photochromic compounds according to any one of [1] to [18] and one or more other photochromic compounds.
[21] The photochromic composition according to [19] or [20], further including a polymerizable compound.
[22] A photochromic article including a cured product obtained by curing the photochromic composition according to [21].
[23] The photochromic article according to [22], including a substrate and a photochromic layer which is the cured product.
[24] The photochromic article according to [22] or [23], which is a spectacle lens.
[25] The photochromic article according to [22] or [23], which is a goggle lens, a visor part of a sun visor or a shield component of a helmet.
[26] Spectacles including the spectacle lens according to [24] .

Two or more of various aspects and various forms described in this specification can be combined in arbitrary combinations.

The embodiments disclosed herein are only examples in all respects and should not be considered as restrictive. The scope of the present invention is not limited to the above description, but is defined by the scope of claims, and is intended to encompass equivalents to the scope of claims and all modifications within the scope of the claims.

### [Industrial Applicability]

One aspect of the present invention is beneficial in the technical fields of spectacles, goggles, sun visors, helmets and the like.

## Claims

1. A photochromic compound represented by the following General Formula 1:
[C1] 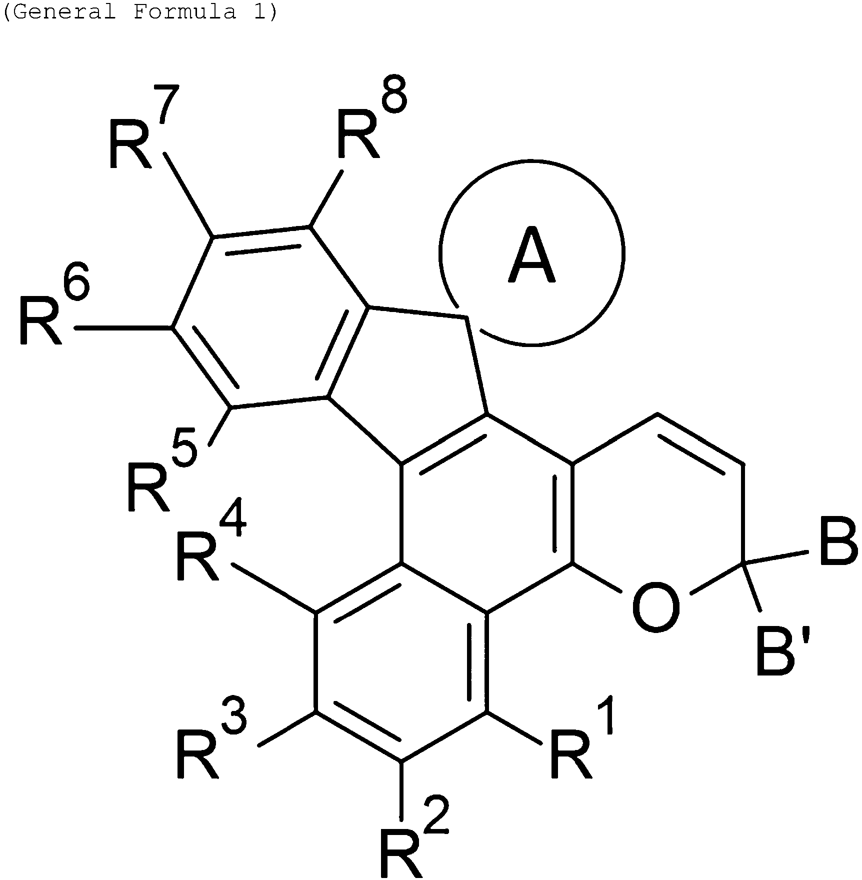 (in General Formula 1,
A represents a ring structure having 3 or more carbon atoms constituting a ring including the carbon atom at position 13 of indeno-fused naphthopyran,
R¹ and R⁴ to R⁸ each independently represent a hydrogen atom or a substituent,
R² represents an electron-donating group (excluding a sulfur-containing electron-donating group),
R³ represents a hydrogen atom or an electron-donating group (excluding a sulfur-containing electron-donating group), and
B and B' each independently represent a substituent).

2. The photochromic compound according to claim 1,
wherein R² represents an alkoxy group and R³ represents a hydrogen atom.

3. The photochromic compound according to claim 1,
wherein R² represents an alkoxy group or a phenoxy group and R³ represents an amino group.

4. The photochromic compound according to claim 3,
wherein R² represents an alkoxy group and R³ represents an amino group.

5. The photochromic compound according to claim 4,
wherein the alkoxy group represented by R² is a methoxy group.

6. The photochromic compound according to any one of claims 3 to 5,
wherein the amino group represented by R³ is a morpholino group or a piperidino group.

7. The photochromic compound according to any one of claims 1 to 6,
wherein R⁷ represents an aryl group.

8. The photochromic compound according to claim 7,
wherein the aryl group represented by R⁷ is a phenyl group.

9. The photochromic compound according to claim 1,
wherein R² and R³ each independently represent an alkoxy group.

10. The photochromic compound according to claim 9,
wherein R² and R³ each represent a methoxy group.

11. The photochromic compound according to claim 9 or 10,
wherein R⁷ represents an aryl group.

12. The photochromic compound according to claim 11,
wherein the aryl group represented by R⁷ is a phenyl group.

13. The photochromic compound according to any one of claims 1 to 12,
wherein the ring structure represented by A is an aliphatic ring.

14. The photochromic compound according to claim 13,
wherein the number of carbon atoms constituting the aliphatic ring is 3 or more and 6 or less.

15. The photochromic compound according to claim 13,
wherein the number of carbon atoms constituting the aliphatic ring is 7 or more and 20 or less.

16. The photochromic compound according to any one of claims 1 to 15,
wherein at least one of B and B' represents a phenyl group having a substituent at a substitution position para to the carbon atom at the position bonding to a pyran ring of indeno-fused naphthopyran.

17. The photochromic compound according to any one of claims 1 to 16,
wherein B and B' each independently represent a phenyl group having an electron-donating group as a substituent at a substitution position para to the carbon atom at the position bonding to a pyran ring of indeno-fused naphthopyran.

18. The photochromic compound according to any one of claims 1 to 17,
wherein R¹, R⁴, R⁵, R⁶ and R⁸ each represent a hydrogen atom.

19. A photochromic composition comprising the photochromic compound according to any one of claims 1 to 18.

20. The photochromic composition according to claim 19, comprising one or more of the photochromic compounds according to any one of claims 1 to 18 and one or more other photochromic compounds.

21. The photochromic composition according to claim 19 or 20, further comprising a polymerizable compound.

22. A photochromic article comprising a cured product obtained by curing the photochromic composition according to claim 21.

23. The photochromic article according to claim 22, comprising a substrate and a photochromic layer which is the cured product.

24. The photochromic article according to claim 22 or 23, which is a spectacle lens.

25. The photochromic article according to claim 22 or 23, which is a goggle lens, a visor part of a sun visor or a shield component of a helmet.

26. Spectacles comprising the spectacle lens according to claim 24.
